# EUROPEAN PATENT APPLICATION

(11) **EP 3 685 831 A1**
(43) Date of publication of application: **29.07.2020**
(21) Application number: 18857890.0
(22) Date of filing: 18.09.2018
(51) Int. Cl.: A61K 31/194, A61K 9/20, A61K 31/295, A61K 45/00, A61K 47/32, A61K 47/34, A61P 7/06

(54) **USE OF FERRIC CITRATE IN PREVENTION AND/OR TREATMENT OF IRON-DEFICIENCY ANEMIA IN HYPERMENORRHEA PATIENT AND/OR PATIENT SUFFERING FROM HYPERMENORRHEA-ASSOCIATED GYNECOLOGIC DISEASE**

(30) Priority: 19.09.2017 JP 2017179533
(71) Applicant: Japan Tobacco Inc., Tokyo 105-8422 (JP)
(72) Inventor: MATSUMOTO, Yu, Tokyo 103-0023 (JP); ARITA, Kojo, Tokyo 103-0023 (JP); MITSUI, Hironori, Tokyo 103-0023 (JP); HANAKI, Koji, Tokyo 103-0023 (JP)
(74) Representative: Vossius & Partner Patentanwälte Rechtsanwälte mbB
(86) International application number: PCT/JP2018/034432
(87) International publication number: WO 2019/059172

(57) **Abstract**

The present invention relates to an agent for preventing and/or treating iron-deficiency anemia in patients with hypermenorrhea and/or patients with hypermenorrhea-associated gynecologic diseases. According to the present invention, an agent containing ferric citrate as an active ingredient for preventing and/or treating iron-deficiency anemia in patients with hypermenorrhea and/or patients with hypermenorrhea-associated gynecologic diseases can be provided.

## Description

### [Technical Field]

The present invention relates to the use of ferric citrate in preventing and/or treating iron-deficiency anemia in patients with hypermenorrhea and/or patients with hypermenorrhea-associated gynecologic diseases.

### [Background Art]

Iron is a metal abundant in the body. Most of iron is used as a component of hemoglobin in erythrocytes and functions to transport oxygen to the whole body. In addition, iron is indispensable for division and proliferation of whole body cells, various metabolism, and the like. In the body, iron is absorbed from the gastrointestinal tract and stored primarily in the liver, but there is no active excretion pathway. The main cause of iron insufficiency (deficiency) is impaired absorption from the gastrointestinal tract, insufficient intake or blood loss. When the iron level in the body decreases, serum ferritin value, which is correlated with the amount of stored iron, first decreases with the "reduction of stored iron". Then "iron deficiency without anemia" (depletion of stored iron but without anemia) is developed, and the transferrin saturation rate (TSAT) decreases due to a decrease in the serum ferritin value, a decrease in serum iron and an increase in total iron binding capacity (TIBC). Furthermore, when iron deficiency progresses, "iron deficiency anemia" develops (Non-patent Document 1). In iron deficiency anemia, anemia symptoms such as palpitation, short breath and the like, and tissue iron deficiency findings such as spoon nail, tongue nipple atrophy, angular cheilitis, swallowing difficulty and the like, allotriophagy, fatigability and the like are observed (Non-patent Document 2), and thus lowering the patients' Quality of Life (QOL).

While the existing oral iron preparations are effective for iron-deficiency anemia excluding absorption disorders after gastrectomy, iron refractory iron deficiency anemia and the like, they cause side effects such as nausea, vomiting, abdominal pain, diarrhea, constipation, urticaria and the like. Gastrointestinal symptoms occur in 20-30% of patients (Non-patent Document 3), and the medication adherence of patients markedly decreases when they experience a strong side effect even once (Non-patent Document 4). Among the gastrointestinal symptoms, the expression rate of nausea and vomiting is high, and the expression rate of nausea and vomiting in the investigation up to the approval of sodium ferrous citrate reached about 13% (Non-patent Document 5). On the other hand, while intravenous iron preparations are advantageous in that they can certainly replenish the necessary iron amount, they sometimes cause headache, fever, nausea, shock symptoms and the like as side effects. In particular, shock symptoms are feared in medical site, and the sale of some of the intravenous iron preparations was discontinued after voluntary recall, given a confirmed increase in the reports of side effects, including hypersensitivity and shock symptoms. There is also a problem of invasiveness associated with the act of injection. Especially in patients with hypermenorrhea, iron supply and hormonal therapy need to be combined. When medication adherence decreases due to the side effects of gastrointestinal symptoms mainly including nausea and vomiting, special attention should be paid since exacerbation of causative disease, risk of pregnancy and the like may also be present.

Hypermenorrhea-associated gynecologic disease mainly includes hysteromyoma, endometriosis, adenomyosis of uterus, endometrial polyp, bicornate uterus and the like. Iron-deficiency anemia associated with hypermenorrhea is sometimes serious, and there is a need for the development of a better therapeutic drug that the patients with hypermenorrhea can safely use in combination with a therapeutic drug for the causative disease (e.g., hormone additive etc.).

On the other hand, it has recently been reported that ferric citrate is useful for the treatment of iron deficiency and anemia in chronic kidney disease (CKD) patients (Patent Documents 1, 2). However, the effectiveness of ferric citrate on iron-deficiency anemia caused by a disease other than CKD has not been confirmed.

### [Document List]

### [Non-patent Documents]

Non-patent Document 1: Bainton DF, Finch CA. Am. J. Med. 1964; 37: 62-70
Non-patent Document 2: Lee GR. Iron deficiency and iron deficiency anemia. In: Wintrobe's Clinical Hematology 10th ed., Lee GR, Foerster J, Lukens J, Paraskevas F, Greer JP, Rodgers GM eds., Williams and Wilkins, Baltimore; 1999: 979-1010
Non-patent Document 3: Takahiro Yano, refractory iron deficiency anemia, Internal medicament 2013; 112: 257-261
Non-patent Document 4: Sadamu Okada, guideline of treatment of iron-deficiency anemia, The Journal of the Japanese Society of Internal Medicament 2010; 99: 1220-1225
Non-patent Document 5: Ferromia (R) tablet 50 mg/ Ferromia (R) granule 8.3% interview forms (IF) of Japanese pharmaceuticals 8th ed., June 2014 rev.

### [Patent documents]

Patent Document 1: National Publication of International Patent Application No. 2015-535209
Patent Document 2: WO 2016/141124

### [SUMMARY OF THE INVENTION]

### [Problems to be Solved by the Invention]

With this background, an oral iron preparation that further reduces the side effects of gastrointestinal symptoms mainly including nausea and vomiting and improves medication adherence is particularly expected to contribute to the improvement of QOL of patients with hypermenorrhea and/or patients with hypermenorrhea-associated gynecologic diseases who are predicted to be subject to a combined hormonal therapy. Thus, the development thereof is strongly demanded.

An object of the present invention is to provide an agent for preventing and/or treating iron-deficiency anemia that causes less side effects such as gastrointestinal symptoms and the like, and can be safely combined with a hormonal therapy and the like in patients with hypermenorrhea and/or patients with hypermenorrhea-associated gynecologic diseases.

### [Means of Solving the Problems]

Under these circumstances, the present inventors have found that ferric citrate is useful for preventing and/or treating iron-deficiency anemia in patients with hypermenorrhea and/or patients with hypermenorrhea-associated gynecologic diseases, and can be used safely in combination with other medicaments (hormonal agent, hemostatic agent, etc.) in patients with hypermenorrhea and/or patients with hypermenorrhea-associated gynecologic diseases, without causing a decrease in efficacy or side effects, which resulted in the completion of the present invention.

Therefore, the present invention provides the following.
[1] An agent for preventing and/or treating iron-deficiency anemia in a patient with hypermenorrhea and/or a patient with a hypermenorrhea-associated gynecologic disease, the agent comprising ferric citrate as an active ingredient.
[2] A method for preventing and/or treating iron-deficiency anemia in a patient with hypermenorrhea and/or a patient with a hypermenorrhea-associated gynecologic disease, the method comprising administering an effective amount of ferric citrate to a patient with hypermenorrhea and/or a patient with a hypermenorrhea-associated gynecologic disease.
[3] Ferric citrate for use in preventing and/or treating iron-deficiency anemia in a patient with hypermenorrhea and/or a patient with a hypermenorrhea-associated gynecologic disease.
[4] Use of ferric citrate for the manufacture of an agent for preventing and/or treating iron-deficiency anemia in a patient with hypermenorrhea and/or a patient with a hypermenorrhea-associated gynecologic disease.
[5] The agent of the above-mentioned [1] wherein the ferric citrate has a molar ratio of ferric ion and citric acid of 1:0.80 to 1:0.92.
[6] The method of the above-mentioned [2] wherein the ferric citrate has a molar ratio of ferric ion and citric acid of 1:0.80 to 1:0.92.
[7] The ferric citrate of the above-mentioned [3] wherein the ferric citrate has a molar ratio of ferric ion and citric acid of 1:0.80 to 1:0.92.
[8] The use of the above-mentioned [4] wherein the ferric citrate has a molar ratio of ferric ion and citric acid of 1:0.80 to 1:0.92.
[9] The agent of the above-mentioned [5] wherein the ferric citrate has a specific surface area of 20 to 45 m²/g.
[10] The method of the above-mentioned [6] wherein the ferric citrate has a specific surface area of 20 to 45 m²/g.
[11] The ferric citrate of the above-mentioned [7] wherein the ferric citrate has a specific surface area of 20 to 45 m²/g.
[12] The use of the above-mentioned [8] wherein the ferric citrate has a specific surface area of 20 to 45 m²/g.
[13] The agent of any of the above-mentioned [1], [5] and [9] wherein the agent is in the form of a tablet.
[14] The method of any of the above-mentioned [2], [6] and [10] wherein the ferric citrate is administered in the form of a tablet.
[15] The ferric citrate of any of the above-mentioned [3], [7] and [11] wherein the ferric citrate is in the form of a tablet.
[16] The use of any of the above-mentioned [4], [8] and [12] wherein the ferric citrate is in the form of a tablet.
[17] The agent of any of the above-mentioned [1], [5], [9] and [13] wherein the patient with hypermenorrhea and/or the patient with a hypermenorrhea-associated gynecologic disease is a female receiving administration of other medicament that affects the amount of menstrual bleeding.
[18] The method of any of the above-mentioned [2], [6], [10] and [14] wherein the patient with hypermenorrhea and/or the patient with a hypermenorrhea-associated gynecologic disease is a female receiving administration of other medicament that affects the amount of menstrual bleeding.
[19] The ferric citrate of any of the above-mentioned [3], [7], [11] and [15] wherein the patient with hypermenorrhea and/or the patient with a hypermenorrhea-associated gynecologic disease is a female receiving administration of other medicament that affects the amount of menstrual bleeding.
[20] The use of any of the above-mentioned [4], [8], [12] and [16] wherein the patient with hypermenorrhea and/or the patient with a hypermenorrhea-associated gynecologic disease is a female receiving administration of other medicament that affects the amount of menstrual bleeding.
[21] The agent of the above-mentioned [17] wherein the other medicament that affects the amount of menstrual bleeding is a hormonal agent or a hemostatic agent.
[22] The method of the above-mentioned [18] wherein the other medicament that affects the amount of menstrual bleeding is a hormonal agent or hemostatic agent.
[23] The ferric citrate of the above-mentioned [19] wherein the other medicament that affects the amount of menstrual bleeding is a hormonal agent or hemostatic agent.
[24] The use of the above-mentioned [20] wherein the other medicament that affects the amount of menstrual bleeding is a hormonal agent or hemostatic agent.
[25] The agent of any of the above-mentioned [1], [5], [9], [13], [17] and [21] comprising 500 mg or 1000 mg of ferric citrate as an effective amount per day.
[26] The method of any of the above-mentioned [2], [6], [10], [14], [18] and [22] wherein a dose of ferric citrate is 500 mg/day or 1000 mg/day.
[27] The ferric citrate of any of the above-mentioned [3], [7], [11], [15], [19] and [23] wherein the ferric citrate is used in an amount of 500 mg/day or 1000 mg/day.
[28] The use of any of the above-mentioned [4], [8], [12], [16], [20] and [24] wherein the ferric citrate is used in an amount of 500 mg/day or 1000 mg/day.
[29] An agent for improving a hemoglobin value in a patient with hypermenorrhea and/or a patient with a hypermenorrhea-associated gynecologic disease, the agent comprising ferric citrate as an active ingredient.
[30] A method for improving a hemoglobin value in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[31] Ferric citrate for use in improving a hemoglobin value in a patient with hypermenorrhea and/or a patient with a hypermenorrhea-associated gynecologic disease.
[32] Use of ferric citrate for the manufacture of an agent for improving a hemoglobin value in a patient with hypermenorrhea and/or a patient with a hypermenorrhea-associated gynecologic disease.
[33] An agent for improving a serum iron level in a patient with hypermenorrhea and/or a patient with a hypermenorrhea-associated gynecologic disease, the agent comprising ferric citrate as an active ingredient.
[34] A method for improving a serum iron level in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[35] Ferric citrate for use in improving a serum iron level in a patient with hypermenorrhea and/or a patient with a hypermenorrhea-associated gynecologic disease.
[36] Use of ferric citrate for the manufacture of an agent for improving a serum iron level in a patient with hypermenorrhea and/or a patient with a hypermenorrhea-associated gynecologic disease.
[37] An agent for improving a serum ferritin value in a patient with hypermenorrhea and/or a patient with a hypermenorrhea-associated gynecologic disease, the agent comprising ferric citrate as an active ingredient.
[38] A method for improving a serum ferritin value in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[39] Ferric citrate for use in improving a serum ferritin value in a patient with hypermenorrhea and/or a patient with a hypermenorrhea-associated gynecologic disease.
[40] Use of ferric citrate for the manufacture of an agent for improving a serum ferritin value in a patient with hypermenorrhea and/or a patient with a hypermenorrhea-associated gynecologic disease.
[41] An agent for improving total iron-binding capacity (TIBC) in a patient with hypermenorrhea and/or a patient with a hypermenorrhea-associated gynecologic disease, the agent comprising ferric citrate as an active ingredient.
[42] A method for improving total iron-binding capacity (TIBC) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[43] Ferric citrate for use in improving total iron-binding capacity (TIBC) in a patient with hypermenorrhea and/or a patient with a hypermenorrhea-associated gynecologic disease.
[44] Use of ferric citrate for the manufacture of an agent for improving total iron-binding capacity (TIBC) in a patient with hypermenorrhea and/or a patient with a hypermenorrhea-associated gynecologic disease.
[45] An agent for improving a transferrin saturation rate (TSAT) in a patient with hypermenorrhea and/or a patient with a hypermenorrhea-associated gynecologic disease, the agent comprising ferric citrate as an active ingredient.
[46] A method for improving a transferrin saturation rate (TSAT) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[47] Ferric citrate for use in improving a transferrin saturation rate (TSAT) in a patient with hypermenorrhea and/or a patient with a hypermenorrhea-associated gynecologic disease.
[48] Use of ferric citrate for the manufacture of an agent for improving a transferrin saturation rate (TSAT) in a patient with hypermenorrhea and/or a patient with a hypermenorrhea-associated gynecologic disease.
[49] An agent for improving a mean corpuscular volume (MCV) in a patient with hypermenorrhea and/or a patient with a hypermenorrhea-associated gynecologic disease, the agent comprising ferric citrate as an active ingredient.
[50] A method for improving a mean corpuscular volume (MCV) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[51] Ferric citrate for use in improving a mean corpuscular volume (MCV) in a patient with hypermenorrhea and/or a patient with a hypermenorrhea-associated gynecologic disease.
[52] Use of ferric citrate for the manufacture of an agent for improving a mean corpuscular volume (MCV) in a patient with hypermenorrhea and/or a patient with a hypermenorrhea-associated gynecologic disease.
[53] An agent for improving a hepcidin concentration in a patient with hypermenorrhea and/or a patient with a hypermenorrhea-associated gynecologic disease, the agent comprising ferric citrate as an active ingredient.
[54] A method for improving a hepcidin concentration in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[55] Ferric citrate for use in improving a hepcidin concentration in a patient with hypermenorrhea and/or a patient with a hypermenorrhea-associated gynecologic disease.
[56] Use of ferric citrate for the manufacture of an agent for improving a hepcidin concentration in a patient with hypermenorrhea and/or a patient with a hypermenorrhea-associated gynecologic disease.
[57] An agent for improving a serum soluble transferrin receptor concentration (sTfR) in a patient with hypermenorrhea and/or a patient with a hypermenorrhea-associated gynecologic disease, the agent comprising ferric citrate as an active ingredient.
[58] A method for improving a serum soluble transferrin receptor concentration (sTfR) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[59] Ferric citrate for use in improving a serum soluble transferrin receptor concentration (sTfR) in a patient with hypermenorrhea and/or a patient with a hypermenorrhea-associated gynecologic disease.
[60] Use of ferric citrate for the manufacture of an agent for improving a serum soluble transferrin receptor concentration (sTfR) in a patient with hypermenorrhea and/or a patient with a hypermenorrhea-associated gynecologic disease.
[61] An agent for improving a C-terminal FGF23 value (cFGF23) in a patient with hypermenorrhea and/or a patient with a hypermenorrhea-associated gynecologic disease, the agent comprising ferric citrate as an active ingredient.
[62] A method for improving a C-terminal FGF23 value (cFGF23) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[63] Ferric citrate for use in improving a C-terminal FGF23 value (cFGF23) in a patient with hypermenorrhea and/or a patient with a hypermenorrhea-associated gynecologic disease.
[64] Use of ferric citrate for the manufacture of an agent for improving a C-terminal FGF23 value (cFGF23) in a patient with hypermenorrhea and/or a patient with a hypermenorrhea-associated gynecologic disease.
[65] An agent for improving an intact FGF23 value (iFGF23) in a patient with hypermenorrhea and/or a patient with a hypermenorrhea-associated gynecologic disease, the agent comprising ferric citrate as an active ingredient.
[66] A method for improving an intact FGF23 value (iFGF23) in a patient with hypermenorrhea and/or a patient with a hypermenorrhea-associated gynecologic disease, the method comprising administering an effective amount of ferric citrate to the patient with hypermenorrhea and/or the patient with a hypermenorrhea-associated gynecologic disease.
[67] Ferric citrate for use in improving an intact FGF23 value (iFGF23) in a patient with hypermenorrhea and/or a patient with a hypermenorrhea-associated gynecologic disease.
[68] Use of ferric citrate for the manufacture of an agent for improving an intact FGF23 value (iFGF23) in a patient with hypermenorrhea and/or a patient with a hypermenorrhea-associated gynecologic disease.
[69] The agent of any of the above-mentioned [29], [33], [37], [41], [45], [49], [53], [57], [61] and [65] wherein the agent comprises 500 mg or 1000 mg of ferric citrate as an effective amount per day.
[70] The method of any of the above-mentioned [30], [34], [38], [42], [46], [50], [54], [58], [62] and [66] wherein a dose of the ferric citrate is 500 mg/day or 1000 mg/day.
[71] The ferric citrate of any of the above-mentioned [31], [35], [39], [43], [47], [51], [55], [59], [63] and [67] wherein the ferric citrate is used in an amount of 500 mg/day or 1000 mg/day.
[72] The use of ferric citrate of any of the above-mentioned [32], [36], [40], [44], [48], [52], [56], [60], [64] and [68] wherein the ferric citrate is used in an amount of 500 mg/day or 1000 mg/day.
[73] A pharmaceutical composition for preventing and/or treating iron-deficiency anemia in a patient with hypermenorrhea and/or a patient with a hypermenorrhea-associated gynecologic disease, the composition comprising ferric citrate and a pharmaceutically acceptable carrier.
[74] The composition of the above-mentioned [73] wherein the ferric citrate has a molar ratio of ferric ion and citric acid of 1:0.80 to 1:0.92.
[75] The composition of the above-mentioned [74] wherein the ferric citrate has a specific surface area of 20 to 45 m²/g.
[76] The composition of any of the above-mentioned [73] to [75] wherein the pharmaceutically acceptable carrier comprises a poly(vinyl alcohol).acrylic acid.methyl methacrylate copolymer and a poly(vinyl alcohol).polyethylene glycol.graft copolymer.
[77] The composition of any of the above-mentioned [73] to [76] wherein the patient with hypermenorrhea and/or the patient with a hypermenorrhea-associated gynecologic disease is a female receiving administration of other medicament that affects the amount of menstrual bleeding.
[78] The composition of the above-mentioned [77] wherein the other medicament that affects the amount of menstrual bleeding is a hormonal agent or a hemostatic agent.
[79] The composition of any of the above-mentioned [73] to [78] wherein the composition comprises 500 mg or 1000 mg of ferric citrate as an effective amount per day.

### [Effect of the Invention]

According to the present invention, a medicament effective for preventing and/or treating iron-deficiency anemia in patients with hypermenorrhea and/or patients with hypermenorrhea-associated gynecologic diseases (e.g., hysteromyoma, endometriosis, adenomyoses of uterus, endometrial polyp, bicornate uterus) can be provided. According to the present invention, moreover, an agent for improving a hemoglobin value, an agent for improving a serum iron level, an agent for improving a serum ferritin value, an agent for improving total iron-binding capacity (TIBC), an agent for improving a transferrin saturation rate (TSAT), an agent for improving a mean corpuscular volume (MCV), an agent for improving a hepcidin concentration (Hepcidin 25), an agent for improving a serum soluble transferrin receptor concentration (sTfR), an agent for improving a C-terminal FGF23 value (cFGF23), and an agent for improving an intact FGF23 value (iFGF23) in patients with hypermenorrhea and/or patients with hypermenorrhea-associated gynecologic diseases can also be provided. The medicament containing ferric citrate as an active ingredient of the present invention can be expected to be used safely without causing development of side effects or decrease in efficacy even when combined with other medicaments (particularly, a hormonal agent that affects the amount of menstrual bleeding, a hemostatic agent and so on) for treating hypermenorrhea-associated gynecologic diseases (primary diseases of hypermenorrhea) such as hysteromyoma, endometriosis, adenomyoses of uterus, endometrial polyp, and bicornate uterus. Therefore, it is particularly useful for preventing and/or treating iron-deficiency anemia in patients with hypermenorrhea and/or patients with hypermenorrhea-associated gynecologic diseases.

### [Description of Embodiments]

The definitions used in the present invention are as follows.

### 1. Ferric citrate

In the present invention, "ferric citrate" to be used as a medicament active ingredient may be a hydrate or a non-hydrate. The form of "ferric citrate" is not particularly limited.

Ferric citrate to be used in the present invention can be produced by a method known per se, for example, the method described in Examples of WO 2012/091139, or a method analogous thereto.

One embodiment of the ferric citrate to be used in the present invention is highly pure ferric citrate substantially free of beta-iron hydroxide oxide. In the aforementioned highly pure ferric citrate, the content of beta-iron hydroxide oxide is in the range of preferably less than 6% by weight, more preferably 2.5% or less by weight, particularly preferably 1.0% or less by weight, especially preferably 0 to 1% by weight, based on the total weight thereof. In the present specification, "substantially free of beta-iron hydroxide oxide" means that the content of beta-iron hydroxide oxide is within the above range, and "highly pure ferric citrate" is ferric citrate in which the content of beta-iron hydroxide oxide is within the above-mentioned range. The content of beta-iron hydroxide oxide in ferric citrate is not limited and can be calculated, for example, by powder X-ray diffractometry.

In addition, one embodiment of the ferric citrate to be used in the present invention is a complex of ferric ion (Fe(III)) and citric acid, and one embodiment thereof is ferric citrate hydrate represented by a complex represented by the molecular formula Fe•x(C₆H₈O₇)•y(H₂O). In the above-mentioned molecular formula, x is preferably 0.75 to 1.10, more preferably 0.78 to 0.95, particularly preferably 0.80 to 0.92, especially preferably 0.81 to 0.91. In another embodiment, x is preferably 0.75 to 1.15, more preferably 0.80 to 1.10. y is preferably 1.8 to 3.2, more preferably 2.4 to 3.1, particularly preferably 2.7 to 3.1. The molar ratio of ferric ion and citric acid is preferably 1:0.75 to 1:1.10, more preferably 1:0.78 to 1:0.95, particularly preferably 1:0.80 to 1:0.92, especially preferably 1:0.81 to 1:0.91. In another embodiment, the molar ratio of ferric ion and citric acid is preferably 1:0.75 to 1:1.15, more preferably 1:0.80 to 1:1.10. The molar ratio of ferric ion and water is preferably 1:1.8 to 1:3.2, more preferably 1:2.4 to 1:3.1, particularly preferably 1:2.7 to 1:3.1.

In one embodiment of the ferric citrate to be used in the present invention, specific surface area is, for example, 20 m²/g or more, preferably 20 to 45 m²/g, more preferably 20 to 40 m²/g. The specific surface area is not limited and can be measured, for example, by a measurement method of BET surface area by a nitrogen gas adsorption method (relative pressure: 0.05 to 0.3).

One embodiment of the ferric citrate to be used in the present invention is an amorphous form, preferably an amorphous powder form.

One embodiment of the ferric citrate to be used in the present invention shows a dissolution ratio of 80% or more, preferably 85% or more, more preferably 90% or more, especially preferably 95% or more, at an elution time of 15 min in a dissolution test by the Japanese Pharmacopoeia, 15th Edition Dissolution Test Method Paddle Method using the first liquid of the Japanese Pharmacopoeia, 15th Edition dissolution test as a test liquid and a rotating speed of 100 times/min.

With the above-mentioned characteristics, the ferric citrate to be used in the present invention can exhibit superior elution property.

2. An agent for preventing and/or treating iron-deficiency anemia in a patient with hypermenorrhea and/or a patient with a hypermenorrhea-associated gynecologic disease, the agent comprising ferric citrate as an active ingredient, or a pharmaceutical composition for preventing and/or treating iron-deficiency anemia a patient with hypermenorrhea and/or a patient with a hypermenorrhea-associated gynecologic disease, the composition comprising ferric citrate and a pharmaceutically acceptable carrier

The agent for preventing and/or treating or the pharmaceutical composition containing ferric citrate as an active ingredient of the present invention can be applied in vivo to any individual of human and non-human animals having iron-deficiency anemia. When applied to human, the agent for preventing and/or treating or the pharmaceutical composition can be provided in the form of a preparation explained below. When applied to a non-human animal, the non-human animal to be targeted is not limited, and is preferably, for example, mouse, rat, hamster, guinea pig, rabbit, cat, dog, swine, bovine, horse, sheep or monkey.

Iron-deficiency anemia can be prevented and/or treated (improved) by applying the agent for preventing and/or treating or the pharmaceutical composition of the present invention to the above-mentioned target (patient or test subject). Accordingly, the present invention also provides a method for preventing and/or treating (improving) iron-deficiency anemia comprising administering the agent for preventing and/or treating or the pharmaceutical composition of the present invention to the above-mentioned patient or test subject.

While the agent for preventing and/or treating or the pharmaceutical composition of the present invention does not limit the target, particularly, it can be preferably used for preventing and/or treating (improving) iron-deficiency anemia in patients with hypermenorrhea and/or patients with hypermenorrhea-associated gynecologic diseases. Iron-deficiency anemia is a condition showing a decreased concentration of hemoglobin in blood, a decreased serum ferritin value and decreased serum iron due to a decrease in stored iron in the body, and a decreased transferrin saturation rate (TSAT) due to an increase in the total iron-binding capacity (TIBC). Therefore, the present invention also provides an agent for improving a hemoglobin value, an agent for improving a serum iron level, an agent for improving a serum ferritin value, an agent for improving total iron-binding capacity (TIBC) or an agent for improving a transferrin saturation rate (TSAT), each containing ferric citrate as an active ingredient.

Iron-deficiency anemia can be prevented and/or treated (improved) by the use of the agent for preventing and/or treating or the pharmaceutical composition of the present invention. In the present specification, "preventing and/or treating iron-deficiency anemia" means, for example, though not limited to, increasing the concentration of hemoglobin in blood to the range of 12 g/dL or more, increasing the serum iron level, lowering the total iron-binding capacity (TIBC) to the range of less than 360 µg/dL, increasing the transferrin saturation rate (TSAT) to the range of 16% or more, and/or increasing the serum ferritin value to the range of 12 ng/mL or more.

The agent for preventing and/or treating or the pharmaceutical composition containing ferric citrate as an active ingredient of the present invention itself alone may be administered to a test subject, or can also be provided as a medicament (e.g., preparation) containing the agent for preventing and/or treating or the pharmaceutical composition, ferric citrate and one or more kinds of pharmaceutically acceptable carriers, for example, additives such as excipient, disintegrant, binder, fluidizer, lubricant, preservative, antioxidant, colorant, and sweetening agent, or other materials known to those of ordinary skill in the art, and, if necessary, other drugs.

The present invention also provides use of ferric citrate for the manufacture of an agent for preventing and/or treating (improving) iron-deficiency anemia in patients with hypermenorrhea and/or patients with hypermenorrhea-associated gynecologic diseases.

In the present specification, "pharmaceutically acceptable" means a compound, a material, a composition and/or a dosage form suitable for use in contact with a tissue of a subject (e.g., human) and, within sound medical judgment, commensurates with a reasonable benefit/risk ratio without causing excessive toxicity, stimulation, allergic reaction or complication. Each additive such as carrier should be "acceptable" in the sense that it can coexist with other components of a preparation.

The agent for preventing and/or treating or the pharmaceutical composition containing ferric citrate as an active ingredient of the present invention (to be also referred to as "the preparation of the present invention") can be appropriately provided as a unit dosage form and can be prepared by any method well known in the technical field of drug manufacture. Such method includes a step of mixing ferric citrate and one or more kinds of subcomponents (e.g., additives such as carrier). In general, a preparation is prepared by uniformly and closely mixing an active compound and a finely pulverized solid carrier or a liquid carrier or both, followed by molding of the resultant product as necessary.

The form (dosage form) of the preparation of the present invention is not limited and includes, for example, an oral preparation such as tablet, capsule, granule, powder, troche, syrup, emulsion or suspension. Among the examples, tablet is preferable.

Tablets may be produced by a conventional means, for example, compression or molding, by optionally adding one or more kinds of the above-mentioned subcomponents. Compressed tablet may be prepared by mixing ferric citrate with, if necessary, one or more kinds of pharmaceutically acceptable carriers selected from the group consisting of excipient, disintegrant, binder, fluidizer, lubricant, preservative, antioxidant, colorant and sweetening agent, and compressing the mixture by a suitable machine. Tablets may be coated or scored as necessary and may be formulated, for example, to provide slow or controlled release of ferric citrate contained in the preparation. Tablets may optionally be provided with an enteric coating to achieve release in the gastrointestinal tract other than the stomach.

Examples of the "excipient" to be used in the preparation (tablet) of the present invention include microcrystalline cellulose (CEOLUS (grade: PH-101, PH-102, PH-301, PH-302, KG-802, KG-1000, manufactured by Asahi Kasei Chemicals Corporation), VIVAPUR (grade: 101, 102, 105, 301, 302, manufactured by JRS Pharma), Emcocel (grade: 50M, 90M, manufactured by JRS Pharma)), lactose, partially pregelatinized starch (e.g., Starch 1500G (manufactured by Japan Colorcon), PCS (manufactured by Asahi Kasei Chemicals Corporation)), sucrose, D-mannitol, D-sorbitol, cornstarch, dextrin, carboxymethylcellulose, calcium carboxymethylcellulose, sodium carboxymethyl starch, and gum arabic.

Examples of the "disintegrant" to be used in the preparation (tablet) of the present invention include low-substituted hydroxypropylcellulose (e.g., L-HPC (grade: LH-11, LH-21, LH-22, LH-31, LH-32, LH-B1, manufactured by Shin-Etsu Chemical Co., Ltd.)), crospovidone (e.g., Kollidon (grade: CL, CL-F, CL-SF, CL-M, manufactured by BASF), Polyplasdone (grade: XL, XL-10, INF-10, manufactured by ISP)), carboxymethylcellulose, calcium carboxymethylcellulose, sodium carboxymethylcellulose, sodium carboxymethyl starch, and croscarmellose sodium.

Examples of the "binder" to be used in the preparation (tablet) of the present invention include poly(vinyl alcohol). polyethylene glycol•graft copolymer (e.g., Kollicoat IR (manufactured by BASF)), poly(vinyl alcohol)•acrylic acid• methyl methacrylate copolymer (e.g., POVACOAT Type: F (manufactured by Daido Chemical Corporation)), hydroxypropylcellulose, hypromellose, polyvinylpyrrolidone, sucrose, dextrin, starch, pregelatinized starch, gelatin, sodium carboxymethylcellulose, and gum arabic.

Examples of the "fluidizer" to be used in the preparation (tablet) of the present invention include light anhydrous silicic acid, magnesium stearate (e.g., magnesium stearate (manufactured by Taihei Chemical Industrial Co., Ltd., The Japanese Pharmacopoeia, plant-derived), magnesium stearate (manufactured by Nitto Kasei Kogyo K.K.), and magnesium stearate (manufactured by Mallinckrodt)).

Examples of the "lubricant" to be used in the preparation (tablet) of the present invention include magnesium stearate, calcium stearate (e.g., calcium stearate (manufactured by Taihei Chemical Industrial Co., Ltd., The Japanese Pharmacopoeia, plant-derived), calcium stearate (manufactured by Nitto Kasei Kogyo K.K.), calcium stearate (manufactured by Mallinckrodt)), sodium stearyl fumarate, stearic acid, and talc.

Examples of the "preservative" to be used in the preparation (tablet) of the present invention include ethyl paraoxybenzoate, chlorobutanol, benzyl alcohol, sodium dehydroacetate, and sorbic acid.

Examples of the "antioxidant" to be used in the preparation (tablet) of the present invention include sodium sulfite and ascorbic acid.

Examples of the "colorant" to be used in the preparation (tablet) of the present invention include food colors (e.g., Food Color Red No. 2 or 3, Food Color yellow No. 4 or 5) and β-carotene.

Examples of the "sweetening agent" to be used in the preparation (tablet) of the present invention include saccharin sodium, dipotassium glycyrrhizinate, and aspartame.

Examples of the "coating agent" to be used in the preparation (tablet) of the present invention include hypromellose, hydroxypropylcellulose, and poly(vinyl alcohol). As for the coating agent, other additives such as plasticizer such as macrogols (e.g., macrogol 6000) and triacetin, light shielding agent such as titanium oxide, anti-adhesion agent such as talc may be mixed if necessary and used for coating. If necessary, a premix coating agent combined with other additives such as plasticizer (e.g., macrogols and triacetin) and light shielding agent (e.g., titanium oxide), which is exemplified by Opadry II (manufactured by Colorcon), may also be used.

The preparation (tablet) of the present invention preferably contains a poly(vinyl alcohol)•acrylic acid.methyl methacrylate copolymer and a poly(vinyl alcohol).polyethylene glycol•graft copolymer as pharmaceutically acceptable carriers, and more preferably further contains, in addition to these, crystalline cellulose, low-substituted hydroxypropylcellulose and crospovidone.

The preparation (tablet) of the present invention contains ferric citrate in terms of non-hydrate at a ratio of preferably 70 parts by mass to 90 parts by mass, further preferably 70 parts by mass to 85 parts by mass, particularly preferably 70 parts by mass to 80 parts by mass, per 100 parts by mass of an uncoated tablet obtained by removing water in ferric citrate.

In another embodiment, the preparation (tablet) of the present invention may contain ferric citrate in terms of non-hydrate at a ratio of preferably 70 parts by mass or more, further preferably 80 parts by mass or more, particularly preferably 85 parts by mass or more, per 100 parts by mass of an uncoated tablet obtained by removing water in ferric citrate.

In the present specification, the "uncoated tablet" means an uncoated tablet before coating in cases where the tablet is a coating tablet, and the tablet itself in cases where the tablet is not coated.

As the preparation of the present invention, a tablet containing ferric citrate at a high content can be provided, which in turn enables formation of a small tablet and/or reduction of the number of tablets to be taken and improvement of administration compliance.

The "in terms of non-hydrate" is an amount corresponding to solid ferric citrate. Specifically, it refers to the amount of ferric citrate in the solid content which is obtained by measuring water in ferric citrate by the Karl Fischer method and excluding such water.

Preferable specific examples of the preparation (tablet) of the present invention include, but are not limited to, those described in the Examples and Formulation Examples of WO 2012/005340 and JP-A-2012-31166.

When the preparation of the present invention is used for treating or improving iron-deficiency anemia, an appropriate dose of ferric citrate varies according to patients. Generally, the dose is selected to achieve, at the site of action, a blood concentration that achieves the desired effect without substantially causing harmful side effects. While the dose level selected here is not limited, it depends on, for example, various factors including administration route of ferric citrate, administration time, excretion rate, duration of treatment, other drugs used in combination, and age, sex, body weight, pathology, general health condition, and the past history of the patient.

In vivo administration can be performed by single administration, continuously or intermittently (e.g., divided administration at appropriate intervals) throughout the entire treatment process. Single or multiple administrations can be performed with the dose level and pattern selected by the treating physician. For example, a preferable dose of ferric citrate in terms of non-hydrate in oral administration to an adult patient is typically about 0.25 to 8 g, preferably about 0.25 to 3 g, more preferably about 0.25 to 2 g, particularly preferably about 0.5 to 1.5 g, per day. A preferable specific dose in terms of non-hydrate is 0.25 g, 0.5 g, 0.75 g, 1.0 g, 1.25 g or 1.5 g. The above-mentioned amounts can be administered once or in divided doses before, between, after or together with meal. In particular, administration after meal is preferable, and administration immediately after meal is particularly preferable.

In the present invention, "treatment" means curing or ameliorating a disease or symptom, or suppressing a symptom, and includes "prevention". The "prevention" means to prevent the onset of a disease or condition.

### 3. Patients with hypermenorrhea and/or patients with hypermenorrhea-associated gynecologic diseases

The "patients with hypermenorrhea" to whom the medicament (the agent for preventing and/or treating the disease, or the pharmaceutical composition) of the present invention is administrated to mean patients (female) with symptoms of iron-deficiency anemia due to hypermenorrhea-associated blood loss. The "patients with hypermenorrhea-associated gynecologic diseases" are different from the aforementioned patients with hypermenorrhea and mean patients affected with gynecologic diseases such as hysteromyoma, endometriosis, adenomyoses of uterus, endometrial polyp, and bicornate uterus which are diseases associated with hypermenorrhea. Many of the patients with hypermenorrhea have hypermenorrhea-associated gynecologic diseases, and therefore, many of the "patients with hypermenorrhea and/or patients with hypermenorrhea-associated gynecologic diseases" in the present invention concurrently use other medicaments (e.g., hormonal agent, hemostatic agent) for treating and/or reducing primary diseases and symptoms of hysteromyoma, endometriosis, adenomyoses of uterus, endometrial polyp, bicornate uterus and the like.

Examples of the aforementioned other medicament include hormonal agents such as therapeutic drug for hysteromyoma, therapeutic drug for endometriosis, therapeutic drug for adenomyoses of uterus, therapeutic drug for endometrial polyp, therapeutic drug for bicornate uterus, and therapeutic drug for dysmenorrhea, analgesic, and hemostatic agent. Specifically, examples of the hormonal agent include GnRH agonist, LHRH derivative, GnRH antagonist, LHRH antagonist, estrogen preparation, gestagen preparation, estrogen.gestagen mixed preparation (e.g., norethisterone.ethynylestradiol, levonorgestrel.ethynylestradiol, desogestrel.ethynylestradiol, drospirenone•ethynylestradiol), and danazol. Examples of the analgesic include NSAIDs such as aspirin, acetaminophen, ibuprofen, diclofenac sodium, and loxoprofen sodium. Examples of the hemostatic agent include tranexamic acid, carbazochrome sodium sulfonate hydrate, and Chinese medicament having a hemostatic action (e.g., kyukikyogaito).

In the present invention, the "hormonal therapy" means a treatment method using hormonal agents or medicaments that promote or suppress hormone secretion (these are collectively referred to as the "hormonal agent" in the present specification) for various symptoms. Examples of the hormonal therapy include gestagen therapy, Kaufmann therapy (estrogen/gestagen cyclic therapy), and a treatment method using contraceptive pills, and examples of the hormonal agent to be used include those mentioned above.

### 4. Iron parameters

Healthy humans have about 4 to 5 g of iron stored within their bodies. About 2.5 g of this iron is contained in hemoglobin, which carries oxygen through the blood. Most of the remaining about 1.5 to 2.5 g of iron is contained in iron binding complexes that are present in all cells, but that are more highly concentrated in bone marrow and organs such as the liver and spleen. Of the body's total iron content, about 400 mg is utilized in proteins that use iron for cellular processes such as oxygen storage (myoglobin) or performing energy-producing redox reactions (cytochrome proteins). In addition to the stored iron, a small amount of iron, typically about 3 to 4 mg, circulates through the blood plasma bound to a protein called transferrin. Because of its toxicity, free soluble ferrous iron (iron(II) or Fe²⁺) is typically kept at a low concentration in the body.

A large amount of bleeding due to hypermenorrhea first depletes the stored iron in the body. Because most of the iron utilized by the body is required for hemoglobin, iron-deficiency anemia is the primary clinical symptom of patients with hypermenorrhea and/or patients with hypermenorrhea-associated gynecologic diseases.

There are several markers of systemic iron status that may be measured to determine whether patients with hypermenorrhea and/or patients with hypermenorrhea-associated gynecologic diseases have sufficient iron stores to maintain adequate health. These markers may be of circulating iron stores, iron stored in iron-binding complexes, or both, and these markers are also typically referred to as iron parameters. Iron parameters can include, for example, total iron-binding capacity (TIBC), transferrin saturation rate (TSAT), serum iron level, liver iron levels, spleen iron levels, serum ferritin values, hepcidin, and serum soluble transferrin receptor (sTfR). Of these, total iron-binding capacity (TIBC), transferrin saturation rate (TSAT) and serum iron levels are commonly known as circulating iron stores. The liver iron levels, spleen iron levels, and serum ferritin values are commonly referred to as stored iron or iron stored in iron-binding complexes. Whether the patients with hypermenorrhea and/or patients with hypermenorrhea-associated gynecologic diseases are in an iron-deficiency anemia state can be diagnosed by examining total iron-binding capacity (TIBC), transferrin saturation rate (TSAT), serum iron level, serum ferritin value, hepcidin, serum soluble transferrin receptor (sTfR) and the like from among the above-mentioned iron parameters.

### 5. Blood hemoglobin value (Hb)

The blood hemoglobin value (Hb) means the weight of hemoglobin contained in a given amount of blood. The standard value (normal value) of the blood hemoglobin value (Hb) is 13.1 to 16.6 g/dL for male and 12.1 to 14.6 g/dL for female, and an iron-deficiency anemia state is suspected when the blood hemoglobin value (Hb) is less than 12 g/dL. However, iron-deficiency anemia cannot be diagnosed from blood hemoglobin value (Hb) alone, and it is comprehensively determined from the below-mentioned measurement results of plural items of total iron-binding capacity (TIBC), transferrin saturation rate (TSAT), serum iron level, serum ferritin value and the like.

### 6. Total iron-binding capacity (TIBC)

Total iron-binding capacity (TIBC) is a measure of the blood's capacity to bind iron with the protein transferrin. TIBC is typically measured by drawing a blood sample and measuring the maximum amount of iron that the sample can carry. Thus, TIBC indirectly measures transferrin, which is a protein that transports iron in the blood. The standard value (normal value) of the total iron-binding capacity (TIBC) is 250 to 385 µg/dL for male and 260 to 420 µg/dL for female. When iron becomes insufficient due to bleeding in patients with hypermenorrhea and/or patients with hypermenorrhea-associated gynecologic diseases, iron absorption from the gastrointestinal tract is promoted to eliminate iron deficiency in the body and the amount of transferrin in the blood is increased to carry iron efficiently in an attempt to eliminate an iron deficiency state. Therefore, when patients with hypermenorrhea and/or patients with hypermenorrhea-associated gynecologic diseases are in an iron-deficiency anemia state, the TIBC value becomes high. When the TIBC value is 360 µg/dL or more, iron-deficiency anemia is suspected, and the diagnosis is made in combination with the measurement results of other parameters.

### 7. Transferrin saturation rate (TSAT)

In addition to the stored iron, typically, a small amount of about 3 to 4 mg of iron bound to a protein called transferrin circulates through blood in plasma. Therefore, the serum iron level can be expressed by the amount of iron bound to the protein transferrin and circulating in the blood. Transferrin is a glycoprotein produced by the liver which can bind to one or two ferric (iron(III) or Fe³⁺) ions. It is the most common and dynamic carrier of iron in the blood and is therefore an essential component of the body's ability to transport stored iron for use in the whole body. The transferrin saturation rate (TSAT) is measured as a percentage and is calculated by multiplying the ratio of serum iron to the total iron-binding capacity (TIBC) by 100. This value shows how much serum iron actually binds relative to the total amount of transferrin that can bind to iron. The standard value (normal value) of TSAT is about 15 to 50% for male and 12 to 45% (about 30%) for female. When patients with hypermenorrhea and/or patients with hypermenorrhea-associated gynecologic diseases are in an iron-deficiency anemia state, the amount of iron that can be bound by transferrin decreases and thus the TSAT value generally reduces remarkably. When the TSAT value is 16% or less, iron-deficiency anemia is suspected, and the diagnosis is made in combination with the measurement results of other parameters.

### 8. Serum iron level

The serum iron level can be expressed by the amount of iron bound to the protein transferrin and circulating in the blood (that is, the amount of iron contained in serum). The standard value (normal value) of serum iron level is 60 to 210 µg/dL for male and 50 to 170 µg/dL for female. When patients with hypermenorrhea and/or patients with hypermenorrhea-associated gynecologic diseases are in an iron-deficiency anemia state, the serum iron level decreases remarkably.

### 9. Serum ferritin value

Ferritin binds to iron and exists as stored iron in the whole body, for example, liver, spleen and bone marrow. When iron in the serum decreases, ferritin supplies iron to transferrin, and when iron increases, ferritin receives iron from transferrin and stores the iron. Since the serum ferritin value is known to correlate well with the amount of stored iron (1 ng/ml of serum ferritin corresponds to 8 to 10 mg of stored iron), a decrease or increase in the stored iron in the body can be observed by examining changes in the serum ferritin value. The standard value (normal value) of serum ferritin value is 20 to 250 ng/mL for male and 5 to 120 ng/mL for female. When patients with hypermenorrhea and/or patients with hypermenorrhea-associated gynecologic diseases are in an iron-deficiency anemia state, the serum ferritin value decreases remarkably. When the serum ferritin value is less than 12 ng/mL, iron-deficiency anemia is suspected, and the diagnosis is made in combination with the measurement results of other parameters.

### 10. Mean corpuscular volume (MCV)

MCV shows the size of erythrocyte contained in the blood. When the MCV value is low, oxygen delivery to the whole body becomes insufficient and anemia symptoms (microcytic anemia) such as short breath and dizziness easily occur. The standard value (normal value) of MCV is 82.7 to 101.6 fL for male and 79 to 100 fL for female. When the MCV value is less than 80 fL, iron-deficiency anemia is suspected, and the diagnosis is made in combination with the measurement results of other parameters.

### 11. Hepcidin (Hepcidin 25)

Hepcidin is secreted in response to excess iron, and the concentration decreases in an iron deficiency state. Therefore, an increase in the Hepcidin 25 value becomes an index of iron-deficiency anemia treatment. It is known that an increase in the Hepcidin 25 value contributes to the prevention of infection and protection of inflammatory tissues.

### 12. Serum soluble transferrin receptor (sTfR)

Transferrin receptor binds to the iron transport protein transferrin in serum and is said to be a membrane protein essential for uptake of iron into cells, whereas a part thereof is liberated into serum. The major part is released from erythroblast which is the center of erythropoiesis, and the serum soluble transferrin receptor (sTfR) concentration is clinically useful for determining the total amount of erythropoiesis. It is useful for examining the recovery sign of hematopoietic ability in anemia. While sTfR rises in iron-deficiency anemia patients, it is a normal value in the case of anemia due to a chronic disease. Thus, a decrease in sTfR is an index in treating iron-deficiency anemia.

### 13. Fibroblast growth factor (FGF) 23 (C-terminal FGF23 (cFGF23), intact FGF23 (iFGF23))

FGF23 is a hormone that is mainly produced by osteocytes and binds to Klotho-FGF receptor complex to exhibit actions such as lowering the concentrations of phosphorus and 1,25-hydroxylated vitamin D. In an iron deficiency state, transcription of FGF23 is promoted, and decomposition of FGF23 is also considered to be promoted. When the iron deficiency state is improved, reduction of cFGF23 is observed. An increase in FGF23 is known to contribute to an increase in the risk of cardiac failure, and reduction of cFGF23 value is expected to contribute to reduction of the risk of cardiac failure.

### 14. Diagnosis criteria of iron-deficiency anemia in patients with hypermenorrhea and/or patients with hypermenorrhea-associated gynecologic diseases

The Japanese BioIron Society treatment guideline production committee Ed., revised treatment guidelines by the proper use of iron, 3rd ed., 2015 describes the diagnosis criteria of iron-deficiency anemia shown in the following Table 1. In the present specification, patients with hypermenorrhea and/or patients with hypermenorrhea-associated gynecologic diseases were also diagnosed according to the diagnosis criteria of Table 1, and subjects to be tested who are in an iron-deficiency anemia state were selected.

**[Table 1]**

| | Hb (g/dL) | TIBC (µg/dL) | Serum ferritin (ng/mL) |
|---|---|---|---|
| Iron-deficiency anemia | <12 | ≥360 | <12 |
| Iron deficiency without anemia | ≥12 | ≥360 or <360 | <12 |
| Normal | ≥12 | <360 | ≥12 |

As shown in the Examples described below, the preparation of the present invention was confirmed to be effective for preventing and/or treating iron-deficiency anemia in patients with hypermenorrhea and/or patients with hypermenorrhea-associated gynecologic diseases. To be specific, regardless of the amount of dose, administration of the preparation of the present invention improved all of the hemoglobin value, serum iron level, serum ferritin value, total iron-binding capacity (TIBC) and transferrin saturation rate (TSAT) in patients with hypermenorrhea and/or patients with hypermenorrhea-associated gynecologic diseases who are in an iron-deficiency anemia state. It was also confirmed that the preparation can be used safely without causing development of side effects or lowering efficacy even when combined with other medicaments (particularly, hormonal agent, hemostatic agent etc. that affect the amount of menstrual bleeding) for treating hysteromyoma, endometriosis, adenomyoses of uterus, endometrial polyp, bicornate uterus and the like which are hypermenorrhea-associated gynecologic diseases.

### [Example]

Hereinafter, the present invention is explained in detail on the basis of Examples, but the present invention is not limited thereto.

### (Example 1)

### Seven-Week administration test of ferric citrate (development code: JTT-751) to iron-deficiency anemia patients (phase II clinical trial)

### (Clinical trial design)

This test was a three-stage safety and efficacy clinical trial at multi-facilities. The first stage was a pre-observation period (Day -40 to Day -1), the second stage was an administration period (Day 1 to Day 48) (efficacy and safety evaluation period), and the third stage was a post-observation period (Day 49 to Day 63) (efficacy and safety evaluation period). The safety evaluation and efficacy evaluation were performed by multi-facility collaboration, placebo control, random allocation, double-blind trial, and parallel group comparison test.

The observation start date is set after the date of an acquisition of the written-agreement from the test subject and before the date of Day -40 to Day -15 (i.e. 39 days to 14 days before the day before the administration start of the trial drug (Week 0)). On the observation start date, the following examination for selection of the test subjects (Table 2) is performed and the qualification is confirmed.

**[Table 2]**

| Examination items | | Note |
|---|---|---|
| Confirmation of qualification of test subject | | In the case of pre-menopausal female, Menstruation Day 8^{*3} is taken as the base date and the examination is performed within ± four days. In the case of pre-menopausal female whose menstruation has been stopped due to a treatment, no visit rule due to menstruation is applied. |
| Test subject background | | |
| Pregnancy test^{*1} | | |
| Past history, complication | | |
| Primary disease | | |
| Simple measurement of Hb value | | |
| Investigation of medicament and therapy | | |
| Erythrocyte-related examination, iron-related examination ^{*2} | | |
| Subjective symptoms, objective findings | | |
| Vital signs | Blood pressure, pulse rate, body temperature | |
| Clinical examination | Hematological inspection | |
| | Blood biochemical examination | |
| | Blood coagulation-based test | |
| | Urine test | |
| Menstrual condition (pre-menopausal female) | | |
| Time of blood collection (iron-related test) | | |

| | | |
|---|---|---|
| *1: Pregnancy test is not performed on male and female whose menopause (absence of menstruation for 52 weeks or more counting from the beginning of the last menstruation and without accompanying other medical reasons) has been confirmed by interview with Scr Visit. *2: Hepcidin 25 and sTfR are not measured. *3: The day when menstruation began is Menstruation Day 1. | | |

Week 0 refers to a name of the observation day that is one day before the administration start of the trial drug (Day -1). The items in Table 3 below are examined on Week 0.

**[Table 3]**

| Examination items | | Note |
|---|---|---|
| Confirmation of qualification of test subject | | In the case of pre-menopausal female, Menstruation Day 6^{*3} is taken as the base date and the examination is performed within ± two days. In the case of pre-menopausal female whose menstruation has been stopped due to a treatment, no visit rule due to menstruation is applied. |
| Test subject background | | |
| Past history, complication | | |
| Simple measurement of Hb value | | |
| Investigation of medicament and therapy | | |
| Erythrocyte-related examination, iron-related examination | | |
| Subjective symptoms, objective findings | | |
| Vital signs | Blood pressure, pulse rate, body temperature | |
| | Body weight | |
| Standard 12-induced electrocardiogram | | |
| Clinical examination | Hematological inspection | |
| | Blood biochemical examination | |
| | Blood coagulation-based test | |
| | Urine test | |
| Menstrual condition (pre-menopausal female) | | |
| Time of blood collection (iron-related test) | | |
| Measurement of FGF23 | | |

| | | |
|---|---|---|
| *1: The day when menstruation began is Menstruation Day 1. | | |

The observation start date and the following observation dates are based on Week 0. It is to be noted that, the next day of Week 0 is the administration start date of the trial drug, namely Day 1.

Week 3 refers to a name of the observation day that is three weeks after Week 0 (Day 21). The items of Table 4 below are examined on Week 3.

**[Table 4]**

| Examination items | | Note |
|---|---|---|
| Medication compliance condition | | Three weeks (21 days) after Week 0 is taken as the base date and the examination is performed within ± four days. In the case of pre-menopausal female, the examination is performed before the scheduled date of menstruation^{*}. In the case of pre-menopausal female whose menstruation has been stopped due to a treatment, no visit rule due to menstruation is applied. |
| Simple measurement of Hb value | | |
| Investigation of medicament and therapy | | |
| Erythrocyte-related examination, iron-related examination | | |
| Investigation of harmful event | | |
| Subjective symptoms, objective findings | | |
| Vital signs | Blood pressure, pulse rate, body temperature | |
| Clinical examination | Hematological inspection | |
| | Blood biochemical examination | |
| | Blood coagulation-based test | |
| | Urine test | |
| Menstrual condition (pre-menopausal female) | | |
| Time of blood collection (iron-related test) | | |

| | | |
|---|---|---|
| *: The scheduled date of menstruation is calculated from the average periods of the most and second recent menstruation before Week 0. | | |

Week 7 refers to a name of the observation day that is seven weeks after Week 0 (Day 49). The items of Table 5 below are examined on Week 7.

**[Table 5]**

| Examination items | | Note |
|---|---|---|
| Medication compliance condition | | Seven weeks (49 days) after Week 0 is taken as the base date and the examination is performed within ± seven days. In the case of pre-menopausal female, the examination is performed before the scheduled date of menstruation^{*}. In the case of pre-menopausal female whose menstruation has been stopped due to a treatment, no visit rule due to menstruation is applied. |
| Simple measurement of Hb value | | |
| Investigation of medicament and therapy | | |
| Erythrocyte-related examination, iron-related examination | | |
| Investigation of harmful event | | |
| Subjective symptoms, objective findings | | |
| Vital signs | blood pressure, pulse rate, body temperature | |
| | Body weight | |
| Standard 12-induced electrocardiogram | | |
| Clinical examination | Hematological inspection | |
| | Blood biochemical examination | |
| | Blood coagulation-based test | |
| | Urine test | |
| Menstrual condition (pre-menopausal female) | | |
| Time of blood collection (iron-related test) | | |
| Measurement of FGF23 | | |

| | | |
|---|---|---|
| *: The scheduled date of menstruation is calculated from the average periods of the most and the second recent menstruation before Week 0. | | |

F-up Visit refers to a name of the observation day that is nine weeks after Week 0 (Day 63). The items of Table 6 below are examined nine weeks after Week 0.

**[Table 6]**

| Examination items | | Note |
|---|---|---|
| Investigation of medicament and therapy | | Nine weeks (63 days) after Week 0 is taken as the base date and the examination is performed within ± seven days. |
| Erythrocyte-related examination, iron-related examination^{*} | | |
| Investigation of harmful event | | |
| Subjective symptoms, objective findings | | |
| Vital signs | Blood pressure, pulse rate, body temperature | |
| Clinical examination | Hematological inspection | |
| | Blood biochemical examination | |
| | Blood coagulation-based test | |
| | Urine test | |
| Menstrual condition (pre-menopausal female) | | |
| Time of blood collection (iron-related test) | | |

| | | |
|---|---|---|
| *: Hepcidin 25 and sTfR are not measured. | | |

### (Sample collection)

Blood and urine are collected from the test subjects to investigate the efficacy, safety and other parameters. The procedures of sample collection and specimen processing follow "Procedure manual of specimen processing". For confirmation of the selection and discontinuation criteria of test subjects, a minute amount of blood collection by fingerstick is performed according to "Procedure for simple measurement of Hb value".

### (Target test subject)

### Iron-deficiency anemia patients

### (Selection criteria)

Before performing the first inspection of the observation start date, a written-agreement is obtained from those who plan to participate in the clinical trial. Patients satisfying the following criteria are the targets.
(1) Japanese patients who are 20 or more and less than 65 years old at the time of agreement and can visit an outpatient clinic
(2) Patients who have an Hb value (measured using HemoCue(R) Hb 201 DM analyzer in the institute of clinical practice) of 7.0 g/dL or more and less than 11.0 g/dL on both the observation start date and Week 0, and the difference between the two days is 1.0 g/dL or less
(3) Patients with a TIBC of 360 µg/dL or more or a serum ferritin value of less than 12 ng/mL on the observation start date

### (Exclusion criteria)

(1) Patients determined by the principal investigator or subinvestigator to have anemia with a main cause that is other than iron deficiency (e.g. patients having a mean corpuscular volume (MCV) of 85 fL or more on the observation start date)
(2) Patients with a serum phosphorus concentration of less than 2.5 mg/dL on the observation start date
(3) In the case of patients with a bleeding lesion that causes iron deficiency, patients who received unscheduled treatments that clearly affect the bleeding within eight weeks before Week 0 (e.g. in the case of patients taking ethinyl estradiol-levonorgestrel combination, patients whose administration is not based on their dosing regimen for the most recent two cycles before Week 0)
(4) In the case of pre-menopausal female patients, patients with a menstrual cycle of 25 days or more and 35 days or less in either the most or second recent menstruation before Week 0
(5) Patients with liver dysfunction (e.g. patients having an aspartic acid aminotransferase (AST) or alanine aminotransferase (ALT) of 100 IU/L or more on the observation start date), or patients with C-type chronic hepatitis as a complication
(6) Patients with gastrointestinal disorder such as acute peptic ulcer, chronic ulcerative colitis, and regional enteritis (excluding chronic gastritis) as complications
(7) Patients with paroxysmal nocturnal hemoglobinuria as a complication
(8) Patients with a history of hypersensitivity to iron-containing preparations
(9) Patients with a history of gastric or duodenal resection (excluding endoscopic resection such as polypectomy)
(10) Patients with a malignant tumor (including hematologic malignancy) as a complication or patients with a history thereof within five years before the observation start date
(11) Patients with a serious complication in, for example, brain, liver, kidney, heart, lung, digestive organ, blood, endocrine system, metabolic system or psychiatric system
(12) Patients who received an intravenous iron preparation within four weeks before the observation start date
(13) Patients who received a transfusion or donated blood within 12 weeks before the observation start date
(14) Patients who were administrated an erythropoiesis-stimulating factor preparation, anabolic hormone, testosterone enanthate, or mepithiostane within 12 weeks before the observation start date
(15) Patients with a history of a serious drug allergy such as anaphylactic shock
(16) Patients with a history of drug addiction or alcoholism, or patients with drug addiction or alcoholism as a complication
(17) Patients who were administered a different trial drug (or test drug) or treated with a trial instrument (or test instrument) within 12 weeks before the observation start date, or patients who participated in a clinical study involving an intervention (which are medical actions exceeding normal medical treatment and practice for study purpose) and were treated
(18) Patients who were administered JTT-751 in a clinical trial or treated with Riona (registered trademark) Tablets 250 mg in the past
(19) Patients who are pregnant, lactating or possibly pregnant (i.e. in cases where the principal investigator or subinvestigator cannot deny the possibility of being pregnant with a pregnancy test on the observation start date or through an interview on Week 0), or patients who are capable of becoming pregnant and failed to agree to prevent pregnancy by an appropriate method during the period between the acquisition of the written-agreement and nine weeks after Week 0 or examination after discontinuation. In the case of male patients, patients who failed to agree to prevent pregnancy by an appropriate method during the period between the start of the trial drug administration and nine weeks after Week 0 or examination after discontinuation.
(20) Other patients determined to be inappropriate as test subjects by the principal investigator or subinvestigator

### (Trial drug)

Ferric citrate (development code: JTT-751) is administered in the form of a tablet (caplet), and each tablet contains 250 mg of ferric citrate.

### (Dose, administration method, and dosing period of trial drug)

The dose of the JTT-751 tablet is 0 mg/day (placebo), 250 mg/day, 500 mg/day, 1000 mg/day or 1500 mg/day as ferric citrate.

The JTT-751 tablet 250 mg or JTT-751 tablet placebo is orally administered 3 times per day (2 tablets at one time), immediately after every meal. The preparation and number of tablets used for each administration period in each administration group are shown in Table 7 below.

**[Table 7]**

| Placebo group | Preparation used | |
|---|---|---|
| | JTT-751 Tablet placebo | JTT-751 Tablet 250 mg |
| Immediately after breakfast | 2 Tablets | - |
| Immediately after lunch | 2 Tablets | - |
| Immediately after dinner | 2 Tablets | - |
| 250 mg Group | Preparation used | |
| | JTT-751 Tablet placebo | JTT-751 Tablet 250 mg |
| Immediately after breakfast | 1 Tablet | 1 Tablet |
| Immediately after lunch | 2 Tablets | - |
| Immediately after dinner | 2 Tablets | - |
| 500 mg Group | Preparation used | |
| | JTT-751 Tablet placebo | JTT-751 Tablet 250 mg |
| Immediately after breakfast | - | 2 Tablets |
| Immediately after lunch | 2 Tablets | - |
| Immediately after dinner | 2 Tablets | - |
| 1000 mg Group | Preparation used | |
| | JTT-751 Tablet placebo | JTT-751 Tablet 250 mg |
| Immediately after breakfast | - | 2 Tablets |
| Immediately after lunch | 2 Tablets | - |
| Immediately after dinner | - | 2 Tablets |
| 1500 mg Group | Preparation used | |
| | JTT-751 Tablet placebo | JTT-751 Tablet 250 mg |
| Immediately after breakfast | - | 2 Tablets |
| Immediately after lunch | - | 2 Tablets |
| Immediately after dinner | - | 2 Tablets |

The administration period of the trial drug is seven weeks which starts from the next day of the Week 0 visit to the day before the Week 7 visit, provided that the trial drug is not taken on the scheduled Week 3 visit.

### (Prohibited combination medicaments)

Use of the following medicaments is prohibited during the period between the observation start date and nine weeks after Week 0 or at the end of investigation of examination at the time of discontinuation.
(1) Oral iron preparations and intravenous injection iron preparations
(2) Erythropoiesis-stimulating factor preparations
(3) Anabolic hormone, testosterone enanthate, mepitiostane
(4) Pharmaceutical products aiming at improving absorption of an oral iron preparation
(5) Pharmaceutical products aiming at treating hyperphosphatemia
(6) Domestic unapproved drugs and other trial drugs (or test drugs)

### (Prohibited combination therapy)

The following therapies are prohibited during the period between the observation start date and nine weeks after Week 0 or at the end of investigation of examination at the time of discontinuation.
(1) Surgical therapies for bleeding lesions that cause iron deficiency
(2) Transfusions
(3) Blood donations
(4) Supplements mainly aiming at improving iron supply or absorption
(5) Domestic unapproved medical instruments and trial instruments (or test instruments)

### (Restricted medicaments for combination use)

(1) During the period between the observation start date and nine weeks after Week 0 or at the end of investigation of examination at the time of discontinuation, except those specified as prohibited combination medicaments, the patient can use medicaments which have already been administered theretobefore the observation start date, provided that the patient does not start using a new medicament or discontinue using the medicament or change the dosage and usage of the medicament. However, the provision does not apply to use of treating and the like of acute diseases or harmful events. As for medicaments that clearly affect the amount of menstrual bleeding (e.g. estrogen•gestagen mixed preparation, gestagen preparation, gestagen release system, GnRH agonist preparation, and danazol preparation), the patient are not permitted to start using a new medicament or discontinue using the medicament or change the dosage and usage of the medicament.
(2) During the period of administration of the trial drug, simultaneous administration of the following medicaments should be carefully prevented by, for example, setting the longest possible administration intervals.
   1) Thyroid hormone preparation (levothyroxine etc.)
   2) Quinolone-type antibacterial agent (ciprofloxacin etc.)
   3) Tetracycline-type antibiotic (tetracycline etc.)
   4) Cefdinir
   5) Therapeutic drug for anti-Parkinson's disease (benserazide, levodopa etc.)
   6) Eltrombopag olamine
   7) Antacid (aluminum hydroxide gel, synthetic aluminum silicate etc.)

### (Evaluation items related to efficacy)

### (1) Main evaluation item

Amount of change in blood hemoglobin value (Hb) from Week 0 at the end of administration

### (2) Subsidiary evaluation items

1) Erythrocyte-related examination values and amount of change from Week 0 at each observation date
2) Iron-related test values and amount of change from Week 0 at each observation date
3) Achievement rate of target Hb value (female; 12.0 g/dL or more, male; 13.0 g/dL or more) at each observation date
4) Achievement rate of 1.0 g/dL or more and achievement rate of 2.0 g/dL or more in Hb value improvement from Week 0 at each observation date
5) Average daily change of Hb value from Week 0 to each observation date

### (Evaluation items related to safety)

(1) Harmful events, side effects (subjective symptoms, objective findings)
(2) Vital signs (blood pressure, pulse rate, body weight, body temperature)
(3) Standard 12-induced electrocardiogram
(4) Clinical examination (hematological inspection, blood biochemical examination, blood coagulation-based test, urine test)

### (Harmful event)

Harmful event refers to every undesirable medical condition occurring in the test subject who received administration of the trial drug, and does not necessarily refers to only those having a clear causal relationship with the administration of the trial drug. That is, the harmful event refers to any unpreferable or unintended symptom (including abnormality in clinical examination values), condition, or disease that occurs when the trial drug is administered, regardless of whether there is a causal relationship with the trial drug. The principal investigator and subinvestigator consider changes in the following subjective symptoms, objective findings, clinical examination, vital sign and standard 12-induced electrocardiogram as harmful events.
(1) Regarding the subjective symptoms and objective findings, when "expression of new abnormality" was observed between the start of the trial drug administration and nine weeks after Week 0 or at the end of examination after discontinuation as compared to before the start of the trial drug administration.
(2) When the symptoms (complications and the like) of the test subject that had been observed before the start of the trial drug administration become aggravated, and thus during the period between the start of the trial drug administration and nine weeks after Week 0 or at the end of examination after discontinuation, new medical events (addition, increase of concomitant medicament etc.) were developed or considered necessary, or the principal investigator or subinvestigator considered that the aggravation of these symptoms was medical exacerbation.
(3) Regarding clinical examination, vital sign and standard 12-induced electrocardiogram, when the principal investigator or subinvestigator determined that an abnormality newly recognized between the start of the trial drug administration and nine weeks after Week 0 or at the end of examination after discontinuation, or aggravation of abnormality observed from before the start of the trial drug administration was a clinical problem.

The investigation period of the harmful event is from the start of the trial drug administration to nine weeks after Week 0 or at the end of examination after discontinuation.

The severity of the harmful event is determined in three levels (Grade 1, Grade 2, Grade 3 are respectively interpreted as light degree, moderate degree, high degree) by reference to the following criteria.

### <Reference criteria>

Light degree: No problem in activities of daily living
Moderate degree: Reduced or affected activities of daily living
High degree: Disabled activities of daily living, or dead

### (Statistical analysis)

### (1) Efficacy analysis target population

As an efficacy analysis target population, a maximum analysis population (FAS: Full Analysis Set) is determined, and an analysis target population (PPS: Per Protocol Set) composed of cases that are compatible with the clinical trial execution plan is determined. The Hb value will start to increase due to an oral iron preparation in one to two weeks after the administration and accordingly, the cases with less than one week of the trial drug administration were excluded from the FAS analysis target population.

### 1) FAS

The cases where the trial drug is administered one week or more, and an investigation related to efficacy is performed at least one time

### 2) PPS

The cases compatible with the clinical trial execution plan; and those which showed a trial drug patient compliance rate of 75% or more, and were evaluated for efficacy on Week 7 (including discontinued cases that were evaluated on the observation day corresponding to Week 7)

### (2) Safety analysis target population

The safety analysis target population means a case where the trial drug is administered, and an investigation related to safety was performed one or more times.

### (3) Handling of calculation items

The standard values used for analyses related to the amount of change in examination values, calculation of change rate, and change (abnormal value in clinical examination value and the like) are taken as the baseline values (Baseline). The baseline value is an examination value measured before the start of the trial drug administration and at a time point closest to Week 0.

### (4) Analysis method

### 1) Significant level

The significant level of the assay related to the investigation item of efficacy is 5% on both sides. When examining the imbalance between the administration groups, about 15% on both sides is used as a rough standard, and a multiplexing adjustment is not performed.

### 2) Calculation method of description statistics

The frequency counting is performed for the count values to calculate the number of cases and percentage. For the measured values, number (N), mean value (Mean), standard deviation (SD), median value (Median), minimum value (Min), and maximum value (Max) are calculated.

### 3) Breakdown of test subject number

For PPS, FAS and safety analysis target populations, the frequency counting of the number of the agreement examples, the allocation example, the unallocation example, the administration example, the non-administration example, the administration completion example, the administration incomplete example, the clinical trial completion example, the clinical trial incomplete example, the reason for incomplete clinical trial is performed for each administration group, and the breakdown of the test subject number is confirmed. As for the discontinued cases, a list of the discontinued cases is prepared and the contents thereof are studied.

### 4) Test subject background

The analysis target populations are PPS, FAS and safety analysis target populations.

The description statistics of the measured values (age, height, body weight, blood pressure, pulse rate, body temperature) are calculated for each administration group. The frequency counting of the count values (sex, the presence or absence of menopause (for female), the presence or absence of the experience of past iron preparation administration and the administration route thereof, the presence or absence of the past history, the presence or absence of the complication, primary disease) is performed for each administration group.

To confirm an imbalance between the administration groups in the test subject background items, a distribution analysis of the measured values is performed. For count values and category variables, when the number of categories is two, the Fisher's exact test is performed, and when the number of categories is three or more, the chi-square test is performed.

### 5) Efficacy

Using FAS and PPS as the targets, as regards the baseline value of efficacy evaluation items, the description statistics are calculated for each administration group. A category is created for each efficacy evaluation item, and the frequency counting is performed. Also, for the measured values to be stratified in the subpopulation analysis, the frequency counting is performed for the category used in the subpopulation analysis. However, the details of the subpopulation analysis are determined in the statistical analysis plan.

To confirm an imbalance between the administration groups in the baseline of the efficacy evaluation items, the distribution analysis of the measured values is performed. For the count values and the category variables, when the number of categories is two, the Fisher's exact test is performed, and when the number of categories is three or more, the chi-square test is performed. With FAS as the target, the description statistics of the examination values and the amount of change at each observation date are calculated for each administration group.

The mean of each administration group is used as a placebo group (µ1), a 250 mg group (µ2), a 500 mg group (µ3), a 1000 mg group (µ4), a 1500 mg group (µ5). Assuming that the dose response is a monotonous increase, the Williams test (significant level one side 2.5%) is performed against the hypothesis shown in the following, and the effective minimum dose is determined from the placebo group (main analysis).

### (Hypothesis)

Comprehensive null hypothesis: the mean (µ) of each group is the same H0: µ1 = µ2 = µ3 = µ4 = µ5
Alternative hypothesis: at least the mean of the 1500 mg group is larger than the mean of the placebo group H1: µ1 ≤ µ2 ≤ µ3 ≤ µ4 ≤ µ5 (provided that at least one "≤" is "<")

### 6) Safety

Using the analysis target population as a safety analysis target population, the harmful events and the side effects expressed under the test treatment are counted and evaluated. Using the preferred term (PT) and the system organ class (SOC) after MedDRA reading, the safety is evaluated with the number of expression (expression rate) of the harmful events and the side effects as the main index.

The description statistics of the vital sign, standard 12-induced electrocardiogram, clinical examination measured value are calculated, and the frequency counting of the classification value is performed for the count values. When an examination value cannot be obtained for an item, the safety is studied based on the findings and the like of the principal investigator.

As regards the abnormal value conflicting with the "standard of abnormal value related to clinical examination item and the like" determined by the examination trial requester of the abnormal value in the examination values, the number of expressions (expression rate) is calculated for each administration group.

### 7) Other investigation

As for the medication compliance condition, using FAS, PPS and the safety analysis target population as the analysis target populations, the patient compliance rate at each time point is calculated for each administration group and the frequency counting is performed. Using the safety analysis target population as the target, the frequency counting is performed for the number of cases, period and dose of trial drug administration, and the exposure status of the safety analysis population is examined. The period is the actual exposure period excluding the suspension period.

As for the menstrual condition, the frequency counting of the number of hypermenorrhea during the clinical trial period is performed for each administration group. In addition, as regards the menstrual period during the clinical trial period, the description statistics are calculated for each administration group.

### (Evaluation of efficacy)

The blood hemoglobin value (Hb), the serum iron level, the serum ferritin value, the total iron-binding capacity (TIBC) and the transferrin saturation rate (TSAT), and the mean corpuscular volume (MCV) were each examined at the observation start date, Week 0, Week 3, Week 7, the end of administration and the consultation at nine weeks after Week 0; the hepcidin concentration (Hepcidin 25) and the serum soluble transferrin receptor concentration (sTfR) were each examined on Week 0, Week 3 and Week 7 and at the end of administration; and the C-terminal FGF23 value (cFGF23) and the intact FGF23 value (iFGF23) were each examined on Week 0 and Week 7 and at the end of administration. The changes in the blood hemoglobin value (Hb) and the respective parameters by the administration of the JTT-751 tablet are explained in the following based on the examination (measurement) results. Of the results of the Phase II clinical trial in the above-mentioned iron-deficiency anemia patients, the following shows the results in the patients with hypermenorrhea and/or the patients with hypermenorrhea-associated gynecologic diseases.

### (Blood hemoglobin value (Hb))

The amount of change in the blood hemoglobin value (Hb) from the baseline at the end of administering ferric citrate (JTT-751 tablet) and the results of the Williams test are shown in Table 8.

**[Table 8]**

| | | Measured value | | Change | | Williams test | | |
|---|---|---|---|---|---|---|---|---|
| Parameter (unit) | | Base line | End of administration | End of administration | | Mean of change amount from the baseline at the end of administration | | |
| Treatment | N | Mean (SD) | Mean (SD) | Mean (SD) | 95% CI | Mean | JTT-751-placebo | p-value |
| Hb (g/dL) | | | | | | | | |
| Placebo | 21 | 9.10 (1.03) | 8.86 (1.20) | -0.25 (1.39) | -0.88 - 0.38 | -0.25 | - | |
| JTT-751 250 mg | 18 | 9.47 (1.02) | 11.72 (1.02) | 2.25 (0.98) | 1.76 - 2.74 | 2.25 | 2.50 | <. 0001* |
| JTT-751 500 mg | 18 | 8.98 (1.00) | 11.71 (0.95) | 2.73 (1.07) | 2.20 - 3.27 | 2.73 | 2.98 | <. 0001* |
| JTT-751 1000 mg | 19 | 9.66 (0.88) | 12.72 (0.76) | 3.05 (1.01) | 2.57 - 3.54 | 3.05 | 3.30 | <. 0001* |
| JTT-751 1500 mg | 21 | 9.38 (1.07) | 12.75 (1.07) | 3.37 (1.52) | 2.68 - 4.06 | 3.37 | 3.62 | <. 0001* |

According to the results of Table 8, the blood hemoglobin value of the ferric citrate (JTT-751 tablet) administration group was confirmed to significantly increase as compared to the placebo administration group regardless of the dose amount. In addition, in the JTT-751 tablet administration group, the blood hemoglobin value was improved to around the standard value (or the standard value or more). Furthermore, it was also confirmed that the blood hemoglobin value increases along with the dose increase of ferric citrate (JTT-751 tablet).

The profiles of blood hemoglobin value (Hb) at each observation start date, Week 0, Week 3, Week 7, the end of administration, and the consultation at nine weeks after Week 0 in each of the ferric citrate (JTT-751 tablet) administration group and the placebo administration group are shown in Tables 9 to 11.

**[Table 9]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| Hb (g/dL) | | | | | | |
| **Observation start date** | N | 21 | 18 | 18 | 19 | 21 |
| | Mean | 9.28 | 9.49 | 9.24 | 9.81 | 9.56 |
| | SD | 1.08 | 0.95 | 0.94 | 1.04 | 1.10 |
| | Min | 7.5 | 7.7 | 7.5 | 7.9 | 7.7 |
| | Median | 9.30 | 9.55 | 9.30 | 9.90 | 9.40 |
| | Max | 11.7 | 11.0 | 11.0 | 11.5 | 11.4 |
| | | | | | | |
| Week 0 | N | 21 | 18 | 18 | 19 | 21 |
| | Mean | 9.10 | 9.47 | 8.98 | 9.66 | 9.38 |
| | SD | 1.03 | 1.02 | 1.00 | 0.88 | 1.07 |
| | Min | 7.3 | 7.5 | 7.0 | 7.9 | 7.6 |
| | Median | 9.10 | 9.80 | 8.95 | 9.80 | 9.40 |
| | Max | 11.4 | 11.2 | 11.0 | 10.8 | 10.9 |

**[Table 10]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| Hb (g/dL) | | | | | | |
| Week 3 | N | 21 | 18 | 18 | 19 | 21 |
| | Mean | 9.20 | 10.57 | 10.39 | 11.72 | 11.60 |
| | SD | 0.91 | 1.06 | 0.94 | 0.89 | 0.90 |
| | Min | 7.6 | 8.6 | 9.1 | 10.2 | 10.2 |
| | Median | 9.40 | 10.75 | 10.10 | 11.80 | 11.60 |
| | Max | 10.5 | 12.5 | 12.5 | 13.9 | 13.4 |
| | | | | | | |
| Week 7 | N | 20 | 18 | 18 | 19 | 21 |
| | Mean | 9.03 | 11.72 | 11.71 | 12.72 | 12.75 |
| | SD | 0.93 | 1.02 | 0.95 | 0.76 | 1.07 |
| | Min | 7.5 | 9.1 | 10.0 | 10.6 | 10.3 |
| | Median | 9.10 | 11.80 | 11.90 | 12.90 | 13.00 |
| | Max | 10.4 | 13.3 | 13.1 | 13.9 | 14. 4 |

**[Table 11]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| Hb (g/dL) | | | | | | |
| **End of administration** | N | 21 | 18 | 18 | 19 | 21 |
| | Mean | 8. 86 | 11.72 | 11.71 | 12.72 | 12.75 |
| | SD | 1.20 | 1.02 | 0.95 | 0.76 | 1.07 |
| | Min | 5.4 | 9.1 | 10.0 | 10.6 | 10.3 |
| | Median | 9.00 | 11.80 | 11.90 | 12.90 | 13.00 |
| | Max | 10.4 | 13.3 | 13.1 | 13.9 | 14.4 |
| | | | | | | |
| **9 weeks after Week 0** | N | 20 | 18 | 17 | 17 | 20 |
| | Mean | 9.03 | 11.51 | 11.62 | 12.73 | 12.73 |
| | SD | 0.95 | 1.02 | 0.71 | 0.75 | 1.09 |
| | Min | 7.3 | 9.0 | 10.2 | 11.6 | 10.6 |
| | Median | 9.05 | 11.60 | 11.70 | 12.70 | 12.70 |
| | Max | 10.9 | 13.1 | 13.1 | 14.2 | 14.8 |

According to the results of Tables 9 to 11, the time-dependent changes in the blood hemoglobin value were not observed in the placebo administration group. However, in the JTT-751 tablet administration group, the blood hemoglobin value also increased along with the continuous administration of the JTT-751 tablet on Week 0, Week 3 and Week 7. The blood hemoglobin value did not decrease even at nine weeks after Week 0 (two weeks after completion of administration).

### (Serum iron level)

The profiles of the serum iron level at Week 0, Week 3, Week 7, the end of administration and the consultation at nine weeks after Week 0 in each of the ferric citrate (JTT-751 tablet) administration group and placebo administration group are shown in Tables 12 to 13.

**[Table 12]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **Serum iron (ug/dL)** | | | | | | |
| Week 0 | N | 21 | 18 | 17 | 19 | 21 |
| | Mean | - | - | - | - | - |
| | SD | - | - | - | - | - |
| | Min | - | - | - | - | - |
| | Median | - | - | - | - | - |
| | Max | - | - | - | - | - |
| | | | | | | |
| Week 3 | N | 21 | 18 | 18 | 19 | 21 |
| | Mean | -0.1 | 19.9 | 29.6 | 55.5 | 53.1 |
| | SD | 26.1 | 15. 9 | 48.7 | 67.2 | 53.9 |
| | Min | -83 | -5 | -10 | -90 | 5 |
| | Median | 1.0 | 18.5 | 13.5 | 36.0 | 27.0 |
| | Max | 55 | 47 | 205 | 203 | 231 |

**[Table 13]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **Serum iron (ug/dL)** | | | | | | |
| Week 7 | N | 20 | 18 | 18 | 19 | 21 |
| | Mean | 8.4 | 30.4 | 40.1 | 48.3 | 54.4 |
| | SD | 33.5 | 24.2 | 22.8 | 47.7 | 59.5 |
| | Min | -31 | -5 | -1 | -71 | -44 |
| | Median | 1.5 | 25.0 | 42.5 | 52.0 | 46.0 |
| | Max | 109 | 87 | 92 | 142 | 240 |
| | | | | | | |
| **End of administration** | N | 21 | 18 | 18 | 19 | 21 |
| | Mean | 4.0 | 30.4 | 40.1 | 48.3 | 54.4 |
| | SD | 38.4 | 24.2 | 22.8 | 47.7 | 59.5 |
| | Min | -84 | -5 | -1 | -71 | -44 |
| | Median | 1.0 | 25.0 | 42.5 | 52.0 | 46.0 |
| | Max | 109 | 87 | 92 | 142 | 240 |
| | | | | | | |
| **9 weeks after Week 0** | N | 20 | 18 | 17 | 16 | 19 |
| | Mean | -0.7 | 14.3 | 24.6 | 40.3 | 38.1 |
| | SD | 10.4 | 15.9 | 16.8 | 40.2 | 35.0 |
| | Min | -30 | -4 | 3 | -68 | -45 |
| | Median | 2.5 | 13.0 | 18.0 | 38.0 | 34.0 |
| | Max | 14 | 65 | 73 | 105 | 139 |

According to the results of Tables 12 to 13, the time-dependent changes in the serum iron level were not observed in the placebo administration group. However, in the JTT-751 tablet administration group, the serum iron level also increased to a normal value of about 50 µg/dL along with the continuous administration of the JTT-751 tablet on Week 0, Week 3 and Week 7. In the JTT-751 tablet 1000 mg administration group and the JTT-751 tablet 1500 mg administration group, the serum iron level was improved to a normal value on Week 3.

### (Serum ferritin value)

The profiles of the serum ferritin value at Week 0, Week 3, Week 7, the end of administration and the consultation at nine weeks after Week 0 in each of the ferric citrate (JTT-751 tablet) administration group and the placebo administration group are shown in Tables 14 to 15.

**[Table 14]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **Ferritin (ng/mL)** | | | | | | |
| | | | | | | |
| Week 0 | N | 21 | 18 | 18 | 19 | 21 |
| | Mean | - | - | - | - | - |
| | SD | - | - | - | - | - |
| | Min | - | - | - | - | - |
| | Median | - | - | - | - | - |
| | Max | - | - | - | - | - |
| | | | | | | |
| Week 3 | N | 21 | 18 | 18 | 19 | 21 |
| | Mean | -0.68 | 11.16 | 15.26 | 26.96 | 20.43 |
| | SD | 2. 59 | 9.38 | 10.52 | 21.87 | 11.30 |
| | Min | -10.8 | -0.5 | -0.2 | -1.0 | 1.3 |
| | Median | -0.10 | 7.70 | 12.40 | 23.20 | 22.20 |
| | Max | 3.0 | 37.2 | 38.9 | 85.5 | 42.3 |
| | | | | | | |
| Week 7 | N | 20 | 18 | 18 | 19 | 21 |
| | Mean | -0.05 | 11.38 | 16.12 | 17.02 | 17.38 |
| | SD | 1.78 | 5.58 | 8.15 | 9.61 | 8.36 |
| | Min | -2.3 | -1.1 | 0.8 | -1.0 | 0.7 |
| | Median | -0.25 | 11.45 | 14.30 | 16.30 | 16.60 |
| | Max | 6.4 | 19.4 | 35.5 | 40.6 | 39.4 |

**[Table 15]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **Ferritin (ng/mL)** | | | | | | |
| **End of administration** | N | 21 | 18 | 18 | 19 | 21 |
| | Mean | -0.53 | 11.38 | 16.12 | 17.02 | 17.38 |
| | SD | 2.81 | 5.58 | 8.15 | 9.61 | 8.36 |
| | Min | -10.2 | -1.1 | 0.8 | -1.0 | 0.7 |
| | Median | -0.30 | 11.45 | 14.30 | 16.30 | 16.60 |
| | Max | 6.4 | 19.4 | 35.5 | 40.6 | 39.4 |
| | | | | | | |
| **9 weeks after Week 0** | N | 20 | 18 | 18 | 17 | 20 |
| | | | | | | |
| | Mean | -0.11 | 2. 88 | 4.28 | 7.51 | 5.83 |
| | SD | 1.18 | 2.44 | 2.83 | 4.06 | 3.84 |
| | Min | -2.9 | -1.2 | 0.5 | 2.8 | 0.6 |
| | Median | -0.10 | 2.60 | 3.60 | 7.20 | 5.35 |
| | Max | 2.7 | 9.0 | 11.3 | 16.4 | 13.9 |

According to the results of Tables 14 to 15, the time-dependent changes in the serum ferritin value were not observed in the placebo administration group. However, in the JTT-751 tablet administration group, the serum iron level also increased to a normal value of about 12 to 30 ng/mL along with the continuous administration of the JTT-751 tablet from Week 0 to Week 3.

### (Total iron-binding capacity (TIBC))

The profiles from the baseline (value at Week 0) of the total iron-binding capacity (TIBC) at Week 0, Week 3, Week 7, the end of administration and the consultation at nine weeks after Week 0 in each of the ferric citrate (JTT-751 tablet) administration group and the placebo administration group are shown in Tables 16 to 17.

**[Table 16]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| TIBC (ug/dL) | | | | | | |
| Week 0 | N | 21 | 18 | 18 | 19 | 21 |
| | Mean | - | - | - | - | - |
| | SD | - - | - - | - - | - - | - - |
| | Median | - | - | - | - | - |
| | Max | - | - | - | - | - |
| | | | | | | |
| Week 3 | N | 21 | 18 | 18 | 19 | 21 |
| | Mean | 2.2 | -28.3 | -30.9 | -54.1 | -60.7 |
| | SD | 29.9 | 22.4 | 26.7 | 33.0 | 30.7 |
| | Min | -48 | -75 | -90 | -104 | -121 |
| | Median | 10.0 | -24.0 | -28.5 | -54.0 | -70.0 |
| | Max | 56 | 20 | 5 | 14 | 19 |

**[Table 17]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| TIBG (ug/dL) | | | | | | |
| Week 7 | N | 20 | 18 | 18 | 19 | 21 |
| | Mean | -3.1 | -60.1 | -61.7 | -80.4 | -79.7 |
| | SD | 24.0 | 29.7 | 30.7 | 40.6 | 39.9 |
| | Min | -68 | -121 | -122 | -172 | -159 |
| | Median | -1.5 | -62.0 | -49.5 | -70.0 | -84.0 |
| | Max | 27 | -2 | -6 | -6 | 20 |
| **End of administration** | N | 21 | 18 | 18 | 19 | 21 |
| | Mean | 0.8 | -60.1 | -61.7 | -80.4 | -79.7 |
| | SD | 29.2 | 29.7 | 30.7 | 40.6 | 39.9 |
| | Min | -68 | -121 | -122 | -172 | -159 |
| | Median | 0.0 | -62.0 | -49.5 | -70.0 | -84.0 |
| | Max | 77 | -2 | -6 | -6 | 20 |
| **9 weeks after Week 0** | N | 20 | 18 | 18 | 17 | 20 |
| | Mean | -4.5 | -42.8 | -49.6 | -68.7 | -63.0 |
| | SD | 23.1 | 29.5 | 29.6 | 36.4 | 40.7 |
| | Min | -37 | -102 | -109 | -132 | -131 |
| | Median | -3.5 | -39.0 | -49.0 | -75.0 | -68.5 |
| | Max | 49 | 15 | 2 | -5 | 42 |

According to the results of Tables 16 to 17, the time-dependent changes in the total iron-binding capacity (TIBC) were not observed in the placebo administration group. However, in the JTT-751 tablet administration group, the total iron-binding capacity (TIBC) also decreased along with the continuous administration of the JTT-751 tablet on Week 0, Week 3 and Week 7.

### (Transferrin saturation rate (TSAT))

The profiles from the baseline (value at Week 0) of the transferrin saturation rate (TSAT) at Week 0, Week 3, Week 7, the end of administration and the consultation at nine weeks after Week 0 in each of the ferric citrate (JTT-751 tablet) administration group and the placebo administration group are shown in Tables 18 to 19.

**[Table 18]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| TSAT (%) | | | | | | |
| Week 0 | N | 21 | 18 | 18 | 19 | 21 |
| | Mean | - | - | - | - | - |
| | SD | - | - | - | - | - |
| | Min | - | - | - | - | - |
| | Median | - | - | - | - | - |
| | Max | - | - | - | - | - |
| | | | | | | |
| Week 3 | N | 21 | 18 | 18 | 19 | 21 |
| | Mean | 0.10 | 5.26 | 8.57 | 15.56 | 15.59 |
| | SD | 7.11 | 3.94 | 13.64 | 17.48 | 15.47 |
| | Min | -22.9 | -0.9 | -0.9 | -20.1 | 2.4 |
| | Median | 0.30 | 4.85 | 3.60 | 9.90 | 7.90 |
| | Max | 16.0 | 11.6 | 55.3 | 55.1 | 66.5 |
| | | | | | | |
| Week 7 | N | 20 | 18 | 18 | 19 | 21 |
| | Mean | 2.33 | 9.12 | 12.27 | 15.46 | 16.70 |
| | SD | 8.58 | 6.90 | 6.50 | 13.30 | 15.64 |
| | Min | -5.7 | -0.6 | 0.2 | -14.7 | -9.8 |
| | Median | 0.25 | 7.60 | 12.35 | 16.20 | 16.00 |
| | Max | 28.9 | 26.6 | 24.6 | 41.3 | 63.3 |

**[Table 19]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **TSAT (%)** | | | | | | |
| **End of administration** | N | 21 | 18 | 18 | 19 | 21 |
| | Mean | 1.10 | 9.12 | 12.27 | 15.46 | 16.70 |
| | SD | 10.09 | 6.90 | 6.50 | 13.30 | 15.64 |
| | Min | -23.5 | -0.6 | 0.2 | -14.7 | -9.8 |
| | Median | 0.20 | 7. 60 | 12.35 | 16.20 | 16.00 |
| | Max | 28.9 | 26.6 | 24.6 | 41.3 | 63.3 |
| | | | | | | |
| **9 weeks after Week 0** | N | 20 | 18 | 18 | 17 | 20 |
| | Mean | -0.02 | 4.29 | 7.27 | 12.42 | 11.41 |
| | SD | 2.30 | 4.25 | 5.51 | 9.81 | 11.08 |
| | Min | -5.8 | -0.6 | 0.7 | -14.8 | -9.5 |
| | Median | 0.60 | 3.85 | 5.90 | 12.60 | 9.95 |
| | Max | 3.9 | 17.1 | 25.1 | 26.7 | 47.4 |

According to the results of Tables 18 to 19, the time-dependent changes in the transferrin saturation rate (TSAT) were not observed in the placebo administration group. However, in the JTT-751 tablet administration group, the transferrin saturation rate (TSAT) also increased (about 17% increase at maximum) along with the continuous administration of the JTT-751 tablet on Week 0, Week 3 and Week 7.

### (Mean corpuscular volume value (MCV))

The profiles of the mean corpuscular volume value (MCV) at the observation start date, Week 0, Week 3, Week 7, the end of administration and the consultation at nine weeks after Week 0 in the ferric citrate (JTT-751 tablet) administration group and the placebo administration group are shown in Table 20.

**[Table 20]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| MCV (fL) Administration start date | N | 21 | 18 | 18 | 19 | 21 |
| | Mean | 71.8 | 71.5 | 71.9 | 72.5 | 71.0 |
| | SD | 6.4 | 4.6 | 6.7 | 5.7 | 6.0 |
| | Min | 62 | 62 | 59 | 65 | 63 |
| | Median | 73.0 | 73.0 | 72.0 | 71.0 | 69.0 |
| | Max | 81 | 78 | 82 | 81 | 84 |
| Week 0 | N | 21 | 18 | 18 | 19 | 21 |
| | Mean | 71.7 | 70.8 | 70.9 | 72.4 | 70.6 |
| | SD | 5.9 | 4.7 | 6.3 | 5.7 | 6.7 |
| | Min | 61 | 62 | 59 | 64 | 60 |
| | Median | 72.0 | 72.0 | 70.5 | 70.0 | 68.0 |
| | Max | 80 | 79 | 80 | 81 | 84 |
| Week 3 | N | 21 | 18 | 18 | 19 | 21 |
| | Mean | 71.0 | 73.9 | 75.3 | 78.3 | 76.7 |
| | SD | 6.9 | 4.5 | 5.0 | 4.3 | 5.0 |
| | Min | 61 | 66 | 67 | 70 | 68 |
| | Median | 72.0 | 74.0 | 75.0 | 77.0 | 77.0 |
| | Max | 80 | 81 | 83 | 85 | 86 |
| Week 7 | N | 20 | 18 | 18 | 19 | 21 |
| | Mean | 70.3 | 78.4 | 79.8 | 82.5 | 81.4 |
| | SD | 5.9 | 4.4 | 4.5 | 3.9 | 3.7 |
| | Min | 60 | 68 | 71 | 75 | 75 |
| | Median | 70.0 | 79.6 | 81.0 | 82.0 | 81.0 |
| | Max | 80 | 85 | 88 | 89 | 90 |
| End of administration | N | 20 | 18 | 18 | 19 | 21 |
| | Mean | 70.6 | 78.4 | 79.8 | 82.5 | 81.4 |
| | SD | 5.9 | 4.4 | 4.5 | 3.9 | 3.7 |
| | Min | 60 | 68 | 71 | 75 | 75 |
| | Median | 71.0 | 79.5 | 81.0 | 82.0 | 81.0 |
| | Max | 80 | 85 | 88 | 89 | 90 |
| 9 weeks after Week 0 | N | 20 | 18 | 18 | 17 | 20 |
| | Mean | 70.0 | 78.9 | 80.8 | 83.9 | 82.4 |
| | SD | 6.4 | 4.7 | 4.3 | 3.3 | 4.4 |
| | Min | 60 | 67 | 72 | 78 | 74 |
| | Median | 71.0 | 79.0 | 81.0 | 84.0 | 82.5 |
| | Max | 82 | 84 | 87 | 89 | 93 |

According to the results of Table 20, the time-dependent changes in the mean corpuscular volume value (MCV) were not observed in the placebo administration group. However, in the JTT-751 tablet administration group, the mean corpuscular volume value (MCV) increased along with the continuous administration of the JTT-751 tablet on Week 0, Week 3 and Week 7 and showed a value around the normal value at the end of administration.

### (Hepcidin concentration (Hepcidin 25))

The profiles of the Hepcidin 25 concentration at Week 0, Week 3, Week 7 and the end of administration in each of the ferric citrate (JTT-751 tablet) administration group and the placebo administration group are shown in Table 21.

**[Table 21]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| **Hepcidin-25** Week 0 | (pg/mL) | | | | | |
| | N | 21 | 18 | 18 | 19 | 21 |
| | Mean | 1.354 | 0.726 | 0.593 | 0.509 | 0.631 |
| | SD | 2.716 | 0.405 | 0.614 | 0.289 | 0.333 |
| | Min | 0.25 | 0.25 | 0.25 | 0.25 | 0.25 |
| | Median | 0.490 | 0.715 | 0.290 | 0.410 | 0.600 |
| | Max | 9.60 | 1.73 | 2.78 | 1.06 | 1.60 |
| Week 3 | N | 21 | 18 | 18 | 19 | 21 |
| | Mean | 0.565 | 1.289 | 1.839 | 8.623 | 7.813 |
| | SD | 0.292 | 1.566 | 1.938 | 5.738 | 6.757 |
| | Min | 0.25 | 0.25 | 0.25 | 0.25 | 0.54 |
| | Median | 0.470 | 0.695 | 0.895 | 8.430 | 5.940 |
| | Max | 1.09 | 5.70 | 6.38 | 19.10 | 24.60 |
| Week 7 | N | 20 | 18 | 18 | 19 | 21 |
| | Mean | 0.624 | 2.196 | 4.818 | 11.465 | 11.674 |
| | SD | 0.359 | 1.789 | 4.487 | 7.063 | 13.445 |
| | Min | 0.25 | 0.49 | 0.25 | 0.50 | 0.79 |
| | Median | 0.580 | 1.520 | 3.925 | 9.680 | 8.440 |
| | Max | 1.63 | 7.34 | 18.60 | 25.90 | 56.70 |
| **End of administration** | N | 21 | 18 | 18 | 19 | 21 |
| | Mean | 0.623 | 2.196 | 4.818 | 11.465 | 11.674 |
| | SD | 0.350 | 1.789 | 4.487 | 7.063 | 13.445 |
| | Min | 0.25 | 0.49 | 0.25 | 0.50 | 0.79 |
| | Median | 0.610 | 1.520 | 3.925 | 9.680 | 8.440 |
| | Max | 1.63 | 7.34 | 18.60 | 25.90 | 56.70 |

According to the results of Table 21, the time-dependent changes in the Hepcidin 25 concentration were not observed in the placebo administration group. However, in the JTT-751 tablet administration group, the Hepcidin 25 concentration increased along with the continuous administration of the JTT-751 tablet on Week 0, Week 3 and Week 7.

### (Serum soluble transferrin receptor concentration (sTfR))

The profiles of the serum soluble transferrin receptor concentration (sTfR) at Week 0, Week 3, Week 7 and the end of administration in each of the ferric citrate (JTT-751 tablet) administration group and the placebo administration group are shown in Table 22.

**[Table 22]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (M = 18) | JTT-751 1000 mg (N = 19) | JTT-751 1500 mg (N = 21) |
| sTfR (nmol/L) Week 0 | N | 21 | 18 | 18 | 19 | 21 |
| | Mean | 48.72 | 47.33 | 44.49 | 48.35 | 44.80 |
| | SD | 19.75 | 12.17 | 16.53 | 35.08 | 14.76 |
| | Min | 20.7 | 32.5 | 16.8 | 19.8 | 23.5 |
| | Median | 45.90 | 43.90 | 43.40 | 42.10 | 43.00 |
| | Max | 98.0 | 70.9 | 74.7 | 183.3 | 81.9 |
| Week 3 | N | 21 | 18 | 18 | 19 | 21 |
| | Mean | 49.99 | 39.26 | 37.27 | 33.09 | 32.53 |
| | SD | 18.57 | 11.54 | 15.76 | 18.31 | 11.18 |
| | Min | 29.6 | 25.0 | 12.7 | 18.0 | 18.4 |
| | Median | 44.00 | 33.90 | 34.10 | 29.00 | 30.40 |
| | Max | 101.9 | 59.6 | 73.6 | 104.6 | 69.1 |
| Week 7 | N | 20 | 18 | 18 | 19 | 21 |
| | Mean | 50.45 | 29.56 | 25.69 | 22.99 | 21.60 |
| | SD | 20.39 | 9.07 | 8.77 | 9.21 | 5.82 |
| | Min | 26.2 | 17.8 | 10.1 | 13.3 | 13.5 |
| | Median | 44.00 | 26.85 | 24.10 | 18.80 | 20.40 |
| | Max | 114.6 | 46.6 | 48.4 | 53.3 | 37.7 |
| **End of administration** | N | 21 | 18 | 18 | 19 | 21 |
| | Mean | 51.64 | 29.56 | 25.69 | 22.99 | 21.60 |
| | SD | 20.61 | 9.07 | 8.77 | 9.21 | 5.82 |
| | Min | 26.2 | 17.8 | 10.1 | 13.3 | 13.5 |
| | Median | 45.70 | 26.85 | 24.10 | 18.80 | 20.40 |
| | Max | 114.6 | 46.5 | 48.4 | 53.3 | 37.7 |

According to the results of Table 22, the time-dependent changes in the serum soluble transferrin receptor concentration (sTfR) were not observed in the placebo administration group. However, in the JTT-751 tablet administration group, the serum soluble transferrin receptor concentration (sTfR) decreased along with the continuous administration of the JTT-751 tablet on Week 0, Week 3 and Week 7.

### (C-terminal FGF23 value (cFGF23))

The profiles of the C-terminal FGF23 value (cFGF23) at Week 0, Week 7 and the end of administration in each of the ferric citrate (JTT-751 tablet) administration group and the placebo administration group are shown in Table 23.

**[Table 23]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| cFGF23 (RU/mL) Week 0 | N | 21 | 18 | 18 | 19 | 21 |
| | Mean | 888.43 | 779.17 | 1011.06 | 675.16 | 842.52 |
| | SD | 848.53 | 472.18 | 1124.84 | 550.51 | 581.78 |
| | Min | 137.0 | 177.0 | 121.0 | 107.0 | 118.0 |
| | Median | 579.00 | 705.00 | 773.50 | 564.00 | 777.00 |
| | Max | 2950.0 | 1920.0 | 5100.0 | 2250.0 | 2260.0 |
| Week 7 | N | 20 | 18 | 18 | 19 | 21 |
| | Mean | 958.40 | 214.72 | 151.89 | 130.39 | 114.92 |
| | SD | 1023.20 | 180.62 | 106,28 | 156.13 | 56.99 |
| | Min | 127.0 | 69.1 | 53.6 | 49.0 | 48.5 |
| | Median | 579.00 | 158.50 | 127.50 | 100.00 | 94.20 |
| | Max | 3710.0 | 792.0 | 540.0 | 764.0 | 246.0 |
| **End of administration** | N | 21 | 18 | 18 | 19 | 21 |
| | Mean | 930.24 | 214.72 | 151.89 | 130.39 | 114.92 |
| | SD | 1005.61 | 180,62 | 106.28 | 156.13 | 56.99 |
| | Min | 127.0 | 69.1 | 53.6 | 49.0 | 48.5 |
| | Median | 525.00 | 158.60 | 127.50 | 100.00 | 94.20 |
| | Max | 3710.0 | 792.0 | 540.0 | 764.0 | 246.0 |

According to the results of Table 23, the time-dependent changes in the C-terminal FGF23 value (cFGF23) were not observed in the placebo administration group. However, in the JTT-751 tablet administration group, the C-terminal FGF23 value (cFGF23) decreased along with the continuous administration of the JTT-751 tablet.

### (Intact FGF23 value (iFGF23))

The profiles of the intact FGF23 value (iFGF23) at Week 0, Week 7 and the end of administration in each of the ferric citrate (JTT-751 tablet) administration group and the placebo administration group are shown in Table 24.

**[Table 24]**

| **Parameter (unit)** | | | | | | |
|---|---|---|---|---|---|---|
| **Consultation** | **Description statistics** | **Placebo** (N = 21) | JTT-751 250 mg (N = 18) | JTT-751 500 mg (N = 18) | JTT-751 1000 mg (N = 19) | JTT-751 1500 mg (N = 21) |
| iFGF23 (pg/mL) Wook 0 | N | 21 | 18 | 18 | 19 | 21 |
| | Mean | 38.04 | 36.73 | 43.53 | 41.93 | 46.09 |
| | SD | 11.69 | 10.57 | 11.58 | 15.40 | 12.80 |
| | Min | 20.2 | 21.6 | 28.1 | 18.2 | 34.1 |
| | Median | 38.20 | 33.90 | 42.05 | 42.50 | 44.00 |
| | Max | 60.7 | 61.2 | 72.3 | 77.0 | 95.6 |
| Week 7 | N | 20 | 18 | 18 | 19 | 21 |
| | Mean | 36.18 | 34.74 | 41.06 | 33.62 | 36.30 |
| | SD | 11.78 | 10.73 | 18.04 | 11.54 | 9.55 |
| | Min | 21.5 | 15.4 | 22.7 | 17.5 | 23.2 |
| | Median | 32.70 | 33.65 | 38.20 | 30.40 | 35.00 |
| | Max | 60.4 | 50.7 | 89.9 | 58.4 | 64.6 |
| **End of administration** | N | 21 | 18 | 18 | 19 | 21 |
| | Mean | 35.93 | 34.74 | 41.06 | 33.62 | 36.30 |
| | SD | 11.54 | 10.73 | 18.04 | 11.54 | 9.55 |
| | Min | 21.5 | 15.4 | 22.7 | 17.5 | 23.2 |
| | Median | 32.30 | 33.65 | 38.20 | 30.40 | 35.00 |
| | Max | 60.4 | 50.7 | 89,9 | 58.4 | 64.6 |

According to the results of Table 24, the time-dependent changes in the intact FGF23 value (iFGF23) were not observed in the placebo administration group. However, in the JTT-751 tablet 1000 mg administration group and the 1500 mg administration group, the intact FGF23 value (iFGF23) decreased along with the continuous administration of the JTT-751 tablet and improved.

From the above results, it was confirmed that the blood hemoglobin value (Hb), the iron parameters and other parameters (serum iron level, serum ferritin value, total iron-binding capacity (TIBC), transferrin saturation rate (TSAT), mean corpuscular volume (MCV), hepcidin concentration (Hepcidin 25), serum soluble transferrin receptor concentration (sTfR), C-terminal FGF23 value (cFGF23), and intact FGF23 value (iFGF23)) that play an important role in preventing and/or treating iron-deficiency anemia in patients with hypermenorrhea and/or patients with hypermenorrhea-associated gynecologic diseases show a remarkable improving tendency in the ferric citrate (JTT-751 tablet) administration group.

### (Evaluation related to safety)

The test subjects of the above-mentioned clinical trial are iron-deficiency anemia patients. Particularly, the patients with hypermenorrhea-associated gynecologic diseases (hysteromyoma, endometriosis, adenomyoses of uterus, endometrial polyp, bicornate uterus etc.) required a combined use of other medicaments and the like (particularly, medicaments affecting the amount of menstrual bleeding) for treating the primary diseases. In the JTT-751 tablet administration group in the above-mentioned clinical trial, carbazochrome sodium sulfonate (hemostatic agent), norethisterone•ethynylestradiol (low dose pill, therapeutic drug for dysmenorrhea), transamin (hemostatic agent), levonorgestrel.ethynylestradiol (oral contraceptive), desogestrel.ethynylestradiol (oral contraceptive), or drospirenone•ethynylestradiol (therapeutic drug for dysmenorrhea), which are medicaments affecting the amount of menstrual bleeding, was actually used in combination; however, no harmful event caused by the combined use was reported.

According to the above results, it was confirmed that ferric citrate (JTT-751 tablet) can be safely administered to patients with hypermenorrhea and/or patients with hypermenorrhea-associated gynecologic diseases even when combined with a medicament affecting the amount of menstrual bleeding, and can remarkably improve numerical value levels of various iron parameters, blood hemoglobin (Hb), mean corpuscular volume (MCV) and C-terminal FGF23 (cFGF23) without causing a decrease in the efficacy.

### [Industrial Applicability]

According to the present invention, a medicament effective for preventing and/or treating iron-deficiency anemia in patients with hypermenorrhea and/or patients with hypermenorrhea-associated gynecologic diseases can be provided. According to the present invention, moreover, an agent for improving a hemoglobin value, an agent for improving a serum iron level, an agent for improving a serum ferritin value, an agent for improving total iron-binding capacity (TIBC), an agent for improving a transferrin saturation rate (TSAT), an agent for improving a mean corpuscular volume (MCV), an agent for improving a hepcidin concentration (Hepcidin 25), an agent for improving a serum soluble transferrin receptor concentration (sTfR), an agent for improving a C-terminal FGF23 value (cFGF23), and an agent for improving an intact FGF23 value (iFGF23) in patients with hypermenorrhea and/or patients with hypermenorrhea-associated gynecologic diseases can also be provided. The medicament containing ferric citrate as an active ingredient of the present invention can be used safely without causing development of side effects or decrease in efficacy even when combined with other medicaments (particularly, a hormonal agent that affects the amount of menstrual bleeding, a hemostatic agent and so on) for treating hysteromyoma, endometriosis, adenomyoses of uterus, endometrial polyp, and bicornate uterus which are primary diseases of hypermenorrhea. Therefore, it is particularly useful for preventing and/or treating iron-deficiency anemia in patients with hypermenorrhea and/or patients with hypermenorrhea-associated gynecologic diseases for whom medication adherence is important because exacerbation of causative disease, risk of pregnancy and the like may be present.

This application is based on a patent application No. 2017-179533 filed in Japan, the contents of which are incorporated in full herein.

## Claims

1. An agent for preventing and/or treating iron-deficiency anemia in a patient with hypermenorrhea and/or a patient with a hypermenorrhea-associated gynecologic disease, the agent comprising ferric citrate as an active ingredient.

2. The agent according to claim 1 wherein the ferric citrate has a molar ratio of ferric ion and citric acid of 1:0.80 to 1:0.92.

3. The agent according to claim 1 or 2 wherein the agent is in the form of a tablet.

4. The agent according to any one of claims 1 to 3 wherein the patient with hypermenorrhea and/or the patient with a hypermenorrhea-associated gynecologic disease is a female receiving administration of other medicament that affects the amount of menstrual bleeding.

5. The agent according to claim 4 wherein the other medicament that affects the amount of menstrual bleeding is a hormonal agent or a hemostatic agent.

6. The agent according to any one of claims 1 to 5 comprising 500 mg or 1000 mg of ferric citrate as an effective amount per day.

7. An agent for improving a hemoglobin value in a patient with hypermenorrhea and/or a patient with a hypermenorrhea-associated gynecologic disease, the agent comprising ferric citrate as an active ingredient.

8. An agent for improving a serum iron level in a patient with hypermenorrhea and/or a patient with a hypermenorrhea-associated gynecologic disease, the agent comprising ferric citrate as an active ingredient.

9. An agent for improving a serum ferritin value in a patient with hypermenorrhea and/or a patient with a hypermenorrhea-associated gynecologic disease, the agent comprising ferric citrate as an active ingredient.

10. An agent for improving total iron-binding capacity (TIBC) in a patient with hypermenorrhea and/or a patient with a hypermenorrhea-associated gynecologic disease, the agent comprising ferric citrate as an active ingredient.

11. An agent for improving a transferrin saturation rate (TSAT) in a patient with hypermenorrhea and/or a patient with a hypermenorrhea-associated gynecologic disease, the agent comprising ferric citrate as an active ingredient.

12. An agent for improving a mean corpuscular volume (MCV) in a patient with hypermenorrhea and/or a patient with a hypermenorrhea-associated gynecologic disease, the agent comprising ferric citrate as an active ingredient.

13. An agent for improving a hepcidin concentration in a patient with hypermenorrhea and/or a patient with a hypermenorrhea-associated gynecologic disease, the agent comprising ferric citrate as an active ingredient.

14. An agent for improving a serum soluble transferrin receptor concentration (sTfR) in a patient with hypermenorrhea and/or a patient with a hypermenorrhea-associated gynecologic disease, the agent comprising ferric citrate as an active ingredient.

15. An agent for improving a C-terminal FGF23 value (cFGF23) in a patient with hypermenorrhea and/or a patient with a hypermenorrhea-associated gynecologic disease, the agent comprising ferric citrate as an active ingredient.

16. An agent for improving an intact FGF23 value (iFGF23) in a patient with hypermenorrhea and/or a patient with a hypermenorrhea-associated gynecologic disease, the agent comprising ferric citrate as an active ingredient.

17. The agent according to any one of claims 7 to 16 wherein the agent comprises 500 mg or 1000 mg of ferric citrate as an effective amount per day.

18. A pharmaceutical composition for preventing and/or treating iron-deficiency anemia in a patient with hypermenorrhea and/or a patient with a hypermenorrhea-associated gynecologic disease, the composition comprising ferric citrate and a pharmaceutically acceptable carrier.

19. The composition according to claim 18 wherein the ferric citrate is substantially free of beta-iron hydroxide oxide and has a molar ratio of ferric ion and citric acid of 1:0.80 to 1:0.92.

20. The composition according to claim 18 or 19 wherein the pharmaceutically acceptable carrier comprises a poly(vinyl alcohol)•acrylic acid•methyl methacrylate copolymer and a poly(vinyl alcohol)•polyethylene glycol•graft copolymer.

21. The composition according to any one of claims 18 to 20 wherein the patient with hypermenorrhea and/or the patient with a hypermenorrhea-associated gynecologic disease is a female receiving administration of other medicament that affects the amount of menstrual bleeding.

22. The composition according to any one of claims 18 to 21 wherein the composition comprises 500 mg or 1000 mg of ferric citrate as an effective amount per day.
